# EUROPÄISCHE PATENTSCHRIFT

(11) **EP 1 368 488 B1**
(45) Veröffentlichungstag und Bekanntmachung des Hinweises auf die Patenterteilung: **26.11.2008**
(21) Anmeldenummer: 02719974.4
(22) Anmeldetag: 28.02.2002
(51) Int. Cl.: C12Q 1/02, C12Q 1/00, G01N 33/487

(54) **SENSORANORDNUNG, VORRICHTUNG UND VERFAHREN ZUR AMPEROMETRISCHEN UND/ODER POTENTIOMETRISCHEN, PHARMAKOLOGISCHEN WIRKORT- UND/ODER WIRKSTOFFTESTUNG**
SENSOR ARRANGEMENT, DEVICE AND METHOD FOR TESTING ACTIVE SUBSTANCES AND/OR ACTIVE SITES FROM A PHARMACOLOGICAL POINT OF VIEW USING AN AMPEROMETER AND/OR POTENTIOMETER
ENSEMBLE DE DETECTION ET DISPOSITIF ET PROCEDE POUR CONTROLER DES SUBSTANCES ACTIVES ET/OU DES SITES ACTIFS D'UN POINT DE VUE PHARMACOLOGIQUE A L'AIDE D'UN AMPEREMETRE ET/OU D'UN POTENTIOMETRE

(30) Priorität: 15.03.2001 DE 10112505
(43) Veröffentlichungstag der Anmeldung: 10.12.2003
(73) Patentinhaber: Iongate Biosciences GmbH, 65926 Frankfurt (DE)
(72) Erfinder: FENDLER, Klaus, 63110 Rodgau-Niederroden (DE)
(74) Vertreter: Hoffmann, Jörg Peter
(86) Internationale Anmeldenummer: PCT/EP2002/002193
(87) Internationale Veröffentlichungsnummer: WO 2002/074983

(56) Entgegenhaltungen:
- EP-A- 0 441 120
- WO-A-96/38726
- WO-A-97/41425
- DE-A- 19 607 279
- US-A- 5 756 355
- US-A- 5 919 576
- US-A- 5 922 594
- D. D. SCHLERETH: "Characterization of protein monolayers by surface plasmon resonance combined with cyclic voltammetry in situ" JOURNAL OF ELECTROANALYTICAL CHEMISTRY, Bd. 464, 1999, Seiten 198-207, XP002207410

## Beschreibung

Sensoranordnung, Vorrichtung und Verfahren zur amperometrischen und/oder potentiometrischen, pharmakologischen Wirkort- und/oder Wirkstofftestung.

Die Erfindung betrifft eine Sensoranordnung, eine Vorrichtung sowie ein Verfahren zur amperometrischen und/oder potentiometrischen, pharmakologischen Wirkort- und/oder Wirkstofftestung.

Bevor Genussmittel, Medikamente und andere Präparate zur Anwendung bei Mensch, Tier und Pflanze kommen können, müssen im Hinblick auf die Zulassung auf dem Markt bestimmte Kriterien hinsichtlich des Nachweises ihrer Wirksamkeit und/oder ihrer Unbedenklichkeit, also hinsichtlich ihrer Nebenwirkungen, erfüllt werden. Entsprechend sind im Stand der Technik unterschiedliche Verfahren und Messeinrichtungen bekannt, mit denen entsprechende Eigenschaften bestimmter Wirkstoffe quantitativ und qualitativ analysiert und beschrieben werden können.

Andererseits ist es oft wünschenswerte, vorgegebene und bekannte Wirkstoffe an einem bestimmten Wirkort zu applizieren, z.B. an einem bestimmten Protein, an einer Zelle, einem Gewebe oder dergleichen, um die Funktionsweise dieses Wirkortes zu beeinflussen und/oder zu untersuchen.

Übliche Experimente am Gesamtorganismus sind aufgrund der Komplexität der Organismen und ferner aufgrund ethischer und moralischer Umstände oft bedenklich, und die damit erzielten Ergebnisse haben eine nur beschränkte Aussagekraft.

Folglich wurden Verfahren und Einrichtungen entwickelt, mit deren Hilfe eine isolierte Betrachtung der Wirkungsweise zu testender Wirkstoffe auf isolierte Wirkzentren oder eine Untersuchung der Wirkzentren selbst - insbesondere im Rahmen eines Deorphaningprozesses bei unbekannter Funktion des Wirkzentrums - möglich ist. Dabei werden bestimmte Gewebe, isolierte Zellen und/oder andere biologische Einheiten, zum Beispiel Proteine oder dergleichen, in isolierter Art und Weise einer Betrachtung zugänglich gemacht. Es sind zum Beispiel Techniken bekannt, die unter Verwendung von Farbstoffen arbeiten und sich ändernde Bindungsspezifitäten ausnutzen und darstellen. Falls diese Vorgehensweise überhaupt möglich ist, ist sie aber dahingehend nachteilig, dass ihr oft nur ein geringes Auflösungsvermögen und eine geringe Empfindlichkeit dabei aber eine hohe Fehleranfälligkeit zukommen.

Andererseits sind aber auch elektrophysiologische Methoden bekannt, zum Beispiel die sogenannten Patchclamp-Technik oder Voltageclamp-Technik, bei welcher zum Beispiel Zellen isoliert einer elektrophysiologischen Untersuchung zugänglich sind. Bei diesem Vorgehen werden native Zellen unterschiedlichen Bedingungen ausgesetzt und es werden bestimmte elektrische Aktivitäten, die durch die Zellen über die darin enthaltenen Organellen und/oder Proteine oder dergleichen vermittelt werden, gemessen. Trotz der dabei erzielbaren hohen Genauigkeit sind diese bekannten elektrophysiologischen Methoden dahingehend nachteilig, dass sie oft eine nur geringe Reproduzierbarkeit der Ergebnisse liefern, aufgrund ihres hohen messtechnischen Aufwandes und ihrer Störanfälligkeit für einen schnellen, automatisierbaren und/oder breiten Einsatz ungeeignet sind und aufgrund der in der Regel nativen Umgebung der zu untersuchenden Objekte gewisse Probleme bei der Diskriminierung unerwünschter Signalanteile aufweisen.

Die DE 196 07 279 A1 offenbart durch Festkörper unterstützte Membranbiosensoren. Diese bekannten Membranbiosensoren bestehen aus einem Festkörper als Träger, einer Lipiddoppelschicht als Membran und einem zwischen dem Träger und der Lipiddoppelschicht eingebauten Spacer. Die Lipiddoppelschicht weist einen eingelagerten Rezeptor als biologische Einheit auf. Im Bereich der Spacer, also zwischen der Lipiddoppelschicht und dem Festkörperträger und auf der vom Festkörper abgewandten Seite der Lipiddoppelschicht befindet sich ein wässriges Messmedium, wodurch den eingebauten Rezeptoren eine natürliche Umgebung zur Aufrechterhaltung ihrer biologischen Funktion gegeben wird.

Eine andere Sensorform ist in der DE 44 37 274 A1 offenbart. Dort ist ein Festkörperträger vorgesehen, auf welchem eine analythspezifische Schicht aus einem flüssigen, festen oder halbfesten Material bestehend aufgebracht ist. Auf dem Träger selbst sind gegebenenfalls mehrere Elektroden vorgesehen, die in der analythspezifischen Schicht in Kontakt mit dem Träger stehend eingebettet sind. Die analythspezifische Schicht ist ein Materialbereich, in welchen gegebenenfalls auch Polypeptite oder Proteine, Rezeptoren oder dergleichen als biologische Einheiten eingebettet sein können. Bei diesem bekannten Sensor kann zwischen der analythspezifischen Schicht als Primärträger und dem als Sekundärträger aufgefassten Festkörperträger zusätzlich eine elektrische Isolation, zum Beispiel aus Teflon, vorgesehen sein.

Die US-PS 5.922.594 offenbart ein Verfahren zum Herstellen von Bilayer Lipid Membranen. Bei diesem Verfahren werden Vesikel, mizelläre Strukturen oder Liposomen an eine Trägeroberfläche gebunden, welche eine Monoschicht in Form einer SAM-Struktur aufweist. Die Monoschicht kann z. B. von Alkanthiolen gebildet werden. Die Membran der Vesikel oder Liposomen kann auch Membranproteine oder andere biologisch aktive membrangebundene Komponenten aufweisen. Die als Träger dienende Metallelektrode, z. B. aus Gold, ist gegenüber einem vorgesehen wässrigen Messmedium, gegenüber den als Primärträger dienenden Vesikeln oder Liposomen und gegenüber den in der Membran der Vesikel oder Liposomen eingebetteten oder integrierten biologischen Einheiten, z. B. den Membranproteinen und dergleichen, elektrisch nicht isoliert.

Die DE 196 07 279 A1 betrifft eine festkörperunterstützte Biosensormembranstruktur. Über dem dort vorgestellten Biosensor wird eine Lipiddoppelschichtmembran auf einem Metallträger kovalent dadurch verankert, dass die dem Metall zugewandte Lipidkomponente der Lipiddoppelschicht aus einem Thiolipid oder Lipid mit Disulfidbrücken gebildet ist. In die Lipiddoppelschichtmembran sind als biologische Einheiten z. B. Rezeptoren eingebracht. Zwischen dem Trägermaterial und der Lipiddoppelschicht befindet sich auch ein wässriges Messmedium, welches den Spacer zwischen der Lipiddoppelschicht und dem metallischen Träger durchsetzt. Es ist keine elektrische Isolation des Sekundärträgers, nämlich der Elektrodenoberfläche oder Trägeroberfläche, gegenüber dem Messmedium, den Primärträgern, oder den biologischen Einheiten in Form der Rezeptoren gegeben.

Die US-PS 5,756,355 offenbart Lipidmembransensoren, welche von einem Lipid-Bilayer gebildet werden, wobei die einem metallischen Träger zugewandte Lipidmonoschicht von einem Thiolipid gebildet wird. Als biologische Einheiten sind z. B. Rezeptorproteine oder Kanäle vorgesehen. Entsprechend der Struktur des Sensors ist keine elektrische Isolation des metallischen Trägers gegenüber dem Primärträger, den biologischen Einheiten und dem Messmedium.

Die EP 0 441 120 A2 offenbart Biosensoren, welche ein wässriges Kompartiment mittels einer Lipiddoppelschicht unterteilen, wobei eine Lipiddoppelschicht biologischer oder synthetischer Ionenkanäle in Kontakt mit dem wässrigen Medium ausgebildet sind. Zwischen einer vorgesehenen Elektrode einerseits und einem Primärträger in Form der Lipiddoppelschicht, den dort integrierten biologischen Einheiten und dem Messmedium andererseits existiert keine elektrische Isolation.

Die US-PS 5,919,576 offenbart Strukturen für immobilisierte biologische Membranen, wobei hier, ausgehend von einem metallischen Träger, eine Alkanthiolmonoschicht auf dem metallischen Träger verwendet wird, um dort biologische Membranen anzulagern oder aufzubringen. Diese biologische Membranen können z. B. Zellmembranen oder dergleichen sein. Aufgrund der Struktur der Membrananordnung liegt auch hier keine elektrische Isolation des zugrunde liegenden metallischen Trägers zu einem ggf. vorgesehenen Messmedium, den Primärträgern oder den biologischen Einheiten vor.

Der Erfindung liegt die Aufgabe zugrunde, eine Sensoranordnung, eine Vorrichtung sowie ein Verfahren zur amperometrischen und/oder potentiometrischen, pharmakologischen Wirkort- und/oder Wirkstofftestung oder dergleichen zu schaffen, bei welchen auf besonders einfache und gleichwohl zuverlässige Art und Weise eine rasche Wirkort- und/oder Wirkstofftestung, insbesondere im Massenbetrieb, unter vertretbaren Kosten möglich ist.

Die Aufgabe wird bei einer Sensoranordnung erfindungsgemäß durch die kennzeichnenden Merkmalen des Anspruchs 1 gelöst. Ferner wird die Aufgabe bei einer Vorrichtung erfindungsgemäß durch die kennzeichnenden Merkmale des Anspruchs 8 gelöst. Des Weiteren wird die Aufgabe bei einem Verfahren zur amperometrischen und/oder potentiometrischen, pharmakologischen Wirkort- und/oder Wirkstofftestung erfindungsgemäß mit den kennzeichnenden Merkmalen des Anspruchs 11 gelöst.

Vorteilhafte Weiterbildungen der erfindungsgemäßen Sensoranordnung, der Vorrichtung und des Verfahrens sind Gegenstand der jeweiligen abhängigen Unteransprüche.

Bei der erfindungsgemäßen Sensoranordnung für die amperometrische und/oder potentiometrische, pharmakologische Wirkort- und/oder Wirkstofftestung ist ein Sekundärträger vorgesehen, welcher einen elektrisch leitfähigen und festkörperartigen Elektrodenbereich aufweist. Ferner ist eine Mehrzahl Primärträger vorgesehen, welche in unmittelbarer räumlicher Nachbarschaft des Sekundärträgers angeordnet sind und welche zu einer elektrischen Aktion aktivierbare und biologische Einheiten, insbesondere Membranproteine, aufweisen. Darüber hinaus ist ein wässriges Messmedium vorgesehen, in welchem die Primärträger und die Sekundärträger angeordnet sind. Der Elekrodenbereich ist gegenüber dem Messmedium, den Primärträgern und gegenüber den biologischen Einheiten elektrisch isoliert ausgebildet. Als Primärträger sind jeweils eine eukariontische Zelle, eine prokariontische Zelle, ein Bakterium; ein Virus, oder Bestandteile, insbesondere Membranfragmente, oder Verbände davon in nativer Form oder in abgewandelter Form, insbesondere in gereinigter, mikrobiologisch und/oder molekularbiologisch geänderter Form, vorgesehen. Alternativ oder zusätzlich sind als Primärträger jeweils ein Vesikel, ein Liposom oder eine mizelläre Struktur vorgesehen.

Maßgeblicher Bestandteil der erfindungsgemäßen Sensoranordnung ist also eine Sensorelektrodeneinrichtung. Diese Sensorelektrodeneinrichtung zur amperometrischen und/oder potentiometrischen, pharmakologischen Wirkort- und/oder Wirkstofftestung selbst also weist mindestens einen elektrisch leitfähigen Elektrodenbereich auf. Die Sensorelektrodeneinrichtung ist ausgebildet, im Betrieb in einem wässrigen Messmedium angeordnet zu werden. Des Weiteren ist die Sensorelektrodeneinrichtung ausgebildet, eine Mehrzahl oder Vielzahl Primärträger mit zu einer elektrischen Aktion aktivierbaren biologischen Einheiten, insbesondere Membranproteinen oder dergleichen, in unmittelbarer räumlicher Nachbarschaft, insbesondere des Elektrodenbereichs, anzuordnen. Dabei ist zumindest der Elektrodenbereich erfindungsgemäß festkörperartig ausgebildet. Des Weiteren ist dabei erfindungsgemäß der Elektrodenbereich ausgebildet, gegenüber dem vorzusehenden Messmedium und gegenüber den Primärträgern elektrisch isoliert zu sein.

Es ist somit eine Kernidee der vorliegenden Erfindung, zumindest den Elektrodenbereich der erfindungsgemäßen Sensoranordnung und insbesondere der Sensorelektrodeneinrichtung festkörperartig oder festkörperunterstützt auszubilden. Dadurch werden der Sensorelektrodeneinrichtung und insbesondere dem vorgesehenen Elektrodenbereich davon eine besonders hohe mechanische Stabilität gegeben, wodurch ein besonders robuster und störunanfälliger Betrieb im Rahmen der Wirkort- und/oder Wirkstofftestung möglich ist.

Erst durch die Festkörperunterstützung wird eine Aktivierung z.B. von Membranproteinen durch einen Konzentrationssprung möglich. Dies kann insbesondere im Rahmen eines schnellen und/oder kontinuierlichen Lösungsaustauschs erfolgen, wodurch - insbesondere bei amperometrsichen Messungen - ein hoher Signalpegel und somit eine hohe Empfindlichkeit erreichbar sind. Aufgrund der Robustheit durch die Festkörperunterstützung sind auch eine leichtere Handhabung und ein bequemer Einbau möglich.

Ein weiterer Kernaspekt der vorliegenden Erfindung besteht darin, den Elektrodenbereich so auszubilden, dass er im Betrieb gegenüber dem Messmedium.und gegenüber den Primärträgern elektrisch isoliert ausgebildet ist. Durch diese Maßnahme wird erreicht, dass die Sensorelektrodeneinrichtung zum Beispiel als kapazitiv gekoppelte Elektrode eingesetzt werden kann. Dies hat insbesondere im Hinblick auf das Signal-zu-Rauschverhältnis, also im Hinblick auf die Nachweisgenauigkeit erhebliche Vorteile. Des Weiteren ist bei der kapazitiven Kopplung der Elektrodenbereich der Sensorelektrodenanordnung oder -einrichtung an keiner chemischen Umsetzung beteiligt, wie das zum Beispiel bei einer typischen elektrochemischen Halbzelle der Fall wäre.

Als weiterer Kernaspekt der erfindungsgemäßen Sensoranordnung ist die Auswahl der die biologischen Einheiten tragenden Primärträger zu sehen. Die Primärträger können jeweils eukariontische Zellen, prokariontische Zellen, Bakterien, Vieren oder Bestandteile, insbesondere Membranfragmente, oder Verbände davon sein, und zwar in nativer Form oder in abgewandelter Form, insbesondere in gereinigter, molekularbiologisch und/oder mikrobiologisch geänderter Form. Alternativ oder zusätzlich sind als Primärträger Vesikel, Liposomen oder mizelläre Strukturen denkbar.

Bei einer besonders vorteilhaften Ausgestaltungsform der erfindungsgemäßen Sensoranordnung ist es vorgesehen, dass der Elektrodenbereich mindestens eine elektrische leitfähige Elektrode aufweist, dass ein elektrisch isolierender Isolationsbereich vorgesehen ist und dass durch den Isolationsbereich die jeweilige Elektrode vom Messmedium, von den Primärträgern und von den biologischen Einheiten elektrisch iso-liert ist.

Der Elektrodenbereich besitzt also vorteilhafterweise mindestens eine Elektrode. Diese kann zum einen jeweils selbst als mechanisch stabiler Materialbereich ausgebildet sein, insbesondere als Platte, als Draht und/oder dergleichen.

Andererseits kann der Elektrodenbereich einen Träger aufweisen, der insbesondere festkörperartig ausgebildet ist. Dann ist es möglich, dass die Elektrode jeweils als Materialbereich oder - schicht auf einem Oberflächenbereich oder der Oberfläche dieses Trägers ausgebildet ist, insbesondere in zusammenhängender Art und Weise. Dabei ist es dann insbesondere vorgesehen, dass die Elektrode durch die Festkörperunterstützung durch den Träger mechanische Stabilität erlangt. Diese Vorgehensweise hat den Vorteil, dass gegebenenfalls hochwertige Materialien zum Beispiel als dünne Schicht auf dem Träger aufgebracht werden können, so dass sich in betriebswirtschaftlicher Hinsicht die Möglichkeit einer Einwegsensor-elektrodeneinrichtung bietet, die zu erschwinglichen Preisen herstellbar und auf dem Markt verwertbar ist. Gegebenenfalls kann der Träger, insbesondere der Elektrodenbereich, wiederverwendet werden, wobei insbsondere ein erneuerter Isolationsbeereich, z.B. eine neue Thiolschicht, notwendig werden kann.

Vorzugsweise weist die Elektrode mindestens ein metallisches Material auf oder ist aus einem solchen Material gebildet. Dabei wird vorteilhafterweise insbesondere ein chemisch inertes Edelmetall verwendet, vorzugsweise Gold. Denkbar sind insbesondere auch Platin oder Silber.

Andererseits kann es auch vorteilhaft sein, dass die Elektrode nicht oder nicht rein metallisch ausgebildet ist und zum Beispiel mindestens ein leitfähiges Metalloxid aufweist. Es bietet sich zum Beispiel Indiumzinnoxid an, weil dieses gegenüber Strahlung zumindest im sichtbaren Bereich und im UV-Bereich vergleichsweise unempfindlich ist, zumindest im Vergleich zu reinen Metallelektroden.

Der Träger zur Aufnahme der Elektrode weist also vorteilhafterweise ein elektrisch isolierendes Material auf oder ist aus einem solchen gebildet. Des Weiteren oder alternativ ist es von Vorteil, dass das Material des Trägers im Wesentlichen chemisch inert ist. Vorteilhafterweise bietet sich als Material ein Glas oder dergleichen an. Dabei kann die Form die einer Platte oder dergleichen sein. Die chemische Inertheit verhindert eine Veränderung sowohl des Trägers als auch eine Verunreinigung des Messmediums während des Messprozesses. Durch die Wahl eines elektrisch isolierenden Trägers wird gewährleistet, dass sämtliche Messsignale im Wesentlichen aus dem Bereich der Elektrode stammen.

Eine besonders einfache Anordnung der Sensorelektrodeneinrichtung ergibt sich, wenn die Elektrode im Wesentlichen als auf der Oberfläche des Trägers abgeschiedene Materialschicht ausgebildet ist. Es kann sich dabei um eine aufgedampfte oder gesputterte Materialschicht handeln. Die Materialschicht zur Ausbildung der Elektrode hat vorzugsweise eine Schichtstärke von etwa 10 bis 200 nm.

Zwischen der Materialschicht für die Elektrode und der Oberfläche des Trägers kann gegebenenfalls eine Haftschicht von Vorteil sein. Insbesondere beim Aufbringen einer Goldelektrode auf Glas ist eine zwischenliegende Haftschicht aus Chrom oder dergleichen von Vorteil. Die Haftschicht hat vorteilhafterweise eine vergleichsweise geringe Schichtdicke, vorzugsweise von etwa 5 nm.

Zur Ausbildung der kapazitiven Elektrode und der dazu notwendigen Isolation des Elektrodenbereichs von Messmedium und/oder von den Primärträgern ist also vorzugsweise mindestens ein Isolationsbereich ausgebildet, durch welchen zumindest im Betrieb der Elektrodenbereich, insbesondere die Elektrode, im Wesentlichen elektrisch isolierbar ist, insbesondere in Bereichen davon, welche im Betrieb zum mechanischen Konatkt mit dem Messmedium und/ oder mit den Primärträgern vorgesehen sind.

Bei einer weiter bevorzugten Ausführungsform der erfindungsgemäßen Sensoranordnung ist der Isolationsbereich schichtartig ausgebildet. Dabei besteht der Isolationsbereich zumindest zum Teil aus einer Abfolge von Monoschichten, wobei die Monoschichten als spontan selbst organisierende Schichten ausgebildet sind.

Dabei ist es von Vorteil, dass als Unterschicht des Isolationsbereichs eine Schicht aus einer organischen Thioverbindung als unterster und der Elektrode zugewandter Bereich des Isolationsbereichs vorgesehen ist, vorzugsweise aus einem langkettigen Alkanthiol, insbesondere aus Oktadekanthiol. Ferner ist als Oberschicht des Isolationsbereiches eine Schicht aus einer amphiphilen organischen Verbindung, insbesondere aus einem Lipid, als oberster und von der Elektrode abgewandter Bereich oder Oberflächenbereich des Isolationsbereichs vorgesehen.

Es kann also von Vorteil sein, den Isolationsbereich zumindest teilweise schichtartig, insbesondere mehrschichtig, auszubilden. Dadurch werden die Isolationswirkung verstärkt und die Herstellung vereinfacht. Um im Betrieb eine möglichst hohe Rate angelagerter und/oder angeordneter Primärträger im Bereich der Sensorelektrodeneinrichtung zu erhalten, ist es gemäß einer bevorzugten Ausführungsform der erfindungsgemäßen Sensorelektrodeneinrichtung vorgesehen, dass zumindest der Oberflächenbereich des Isolationsbereichs derart abgestimmt ausgebildet ist, dass eine Anlagerung und/oder Anordnung von Primärträgern am Oberflächenbereich des Isolationsbereichs begünstigt wird, insbesondere in mit der Oberfläche der Primärträger kompatibler Art und Weise.

Das bedeutet, dass je nach Oberflächenbeschaffenheit der Primärträger der Oberflächenbereich des Isolationsbereichs der Sensorelektrodeneinrichtung entsprechend angepasst ausgebildet ist, so dass sich die Primärträger begünstigt am Oberflächenbereich des Isolationsbereichs anlagern und dort auch verbleiben.

Im Hinblick auf eine besonders ausgeprägte kapazitive Kopplung der Sensorelektrodeneinrichtung im Messbetrieb ist es vorgesehen, dass der Isolationsbereich zumindest teilweise als Monoschicht, Monolage und/oder als Abfolge davon ausgebildet ist. In diesem Fall ist die auf die Fläche bezogene spezifische elektrische Kapazität der Elektrodengrenzschicht besonders hoch. Besonders einfach gestaltet sich die Anordnung und Ausbildung der erfindungsgemäßen Sensoranordnung, wenn die Schicht oder die Schichten des Isolationsbereichs als sich spontan selbstorganisierende Schichten oder als Self-Assembling-Schichten ausgebildet sind oder werden. Dabei werden in vorteilhafter Art und Weise die Neigung und das Bestreben bestimmter, im Wesentlichen flüssiger oder flüssig gelöster Ausgangsstoffe ausgenutzt, auf einer Oberfläche unter Einfluss der Wechselwirkung mit der Struktur der Oberfläche spontan und selbstorganisierend eine geordnete und/oder schichtartige Struktur auszubilden, die unter bestimmten Umständen und bei bestimmten Stoffklassen zur Ausbildung besonders dünner und gegebenenfalls einlagiger Schichten oder Monoschichten, insbesondere von Molekülen, führt.

Dabei ist es also von Vorteil, wenn als Unterschicht oder als unterster und der Elektrode im Wesentlichen zugewandter Bereich des Isolationsbereichs eine Schicht aus einer organischen Thioverbindung vorgesehen ist. Im Hinblick auf die elektrischen Eigenschaften bietet sich dabei vorzugsweise die Verwendung eines langkettigen Alkanthiols und/oder an. Besonders bevorzugt ist dabei die Zugrundelegung eines C18-Alkans, also Oktadekanthiol.

Bei dieser Vorgehensweise wird ausgenutzt, dass auf bestimmten Edelmetalloberflächen, zum Beispiel Gold, Silber und Platin, aus einer organischen Lösung, welche entsprechende Thioverbindung in Lösung enthält, aufgrund einer spezifischen Wechselwirkung der Thiogruppe mit den Oberflächenatomen der Edelmetallelektrode eine kovalent gebundene Monoschicht auf der Elektrodenoberfläche entstehen kann, die bei entsprechender Geometrie der Thioverbindung eine hexagonal dichte Packung auszubilden vermag, wodurch eine besonders geringe Restleitfähigkeit der Edelmetalloberfläche in Bezug auf das vorzusehende Messmedium realisierbar ist.

Andererseits ist es vorgesehen, dass als Oberschicht oder als oberster und von der Elektrode im Wesentlichen abgewandter Bereich oder Oberflächenbereich des Isolationsbereichs eine Schicht einer amphiphilen organischen Verbindung vorgesehen ist, insbesondere eines Lipids und/oder dergleichen.

Durch dieses Vorgehen wird ebenfalls eine Anordnung und Strukturierung der Oberfläche des Isolationsbereichs erzwungen. Die amphiphilen Verbindungen besitzen zumindest einen Bereich, der im Wesentlichen polar ausgebildet ist, so dass sich im Messmedium, welches insbesondere wässrige Natur besitzt, eine gewisse partielle Lösbarkeit ergibt. Andererseits besitzen amphiphile organische Verbindungen einen unpolaren oder hydrophoben Bereich, dessen Anordnung in einem wässrigen Messmedium energetisch weniger bevorzugt ist. Durch diese Phänomene bildet sich bevorzugt eine Schichtstruktur aus, bei welcher die polaren oder wasserlöslichen Bereiche der amphiphilen Verbindung dem wässrigen Messmedium zugeordnet sind, wogegen sich die unpolaren oder hydrophoben Bereiche der amphiphilen organischen Verbindung vom wässrigen Messmedium abgewandt anordnen. Es kann sich somit eine Monoschicht ausbilden, die insbesondere den Oberflächenbereich des Elektrodenbereichs bildet. Dies geschieht bevorzugt in Kombination mit einer Alkanthiolmonoschicht als Unterschicht, so dass sich als Isolationsbereich im Wesentlichen zumindest teilweise eine Doppelschicht zweier Monoschichten oder Monolagen ergibt.

Die so ausgebildete Abfolge zweier Monoschichten hat gewisse strukturelle Ähnlichkeiten mit bestimmten Membranstrukturen, die aus biologischen Systemen bekannt sind, so dass man der so ausgebildeten Abfolge zweier Monoschichten - nämlich der der Elektrode zugewandten Alkanthiolmonoschicht und der darüber angeordneten Lipidmonoschicht - eine gewisse Membranstruktur zuordnen kann. Aufgrund des zugrundeliegenden Festkörperträgers bezeichnat man diese Membranstruktur auch als festkörperunterstützte Membran SSM (SSM: solid supported membrane). Diese Membranstruktur SSM hat im Hinblick auf die Anordnung und Eigenschaft der erfindungsgemäßen Sensorelektrodeneinrichtung als kapazitiv gekoppelte Elektrode besonders günstige Eigenschaften.

Insbesondere weist derjenige Bereich, welcher durch den die Elektrode isolierenden und/oder abdeckenden Bereich des Isolationsbereichs definiert wird, die eben beschriebene Membranstruktur in vorteilhafter Weise auf. Dabei ist es von Vorteil, dass diese Membranstruktur zumindest teilweise eine spezifische elektrische Leitfähigkeit von etwa Gₘ ≈ 1 - 100 nS/cm² besitzt. Des Weiteren ergibt sich in vorteilhafter Weise eine spezifische elektrische Kapazität von etwa Cₘ ≈ 10 - 1000 nF/cm². Schließlich ist alternativ oder ergänzend eine Fläche für die Membranstruktur von etwa A ≈ 0,1 - 50 mm² vorgesehen.

Bei einer weiteren vorteilhaften Ausführungsform der erfindungsgemäßen Sensoranordnung ist es vorgesehen, dass der die Elektrode isolierende und abdeckende Bereich des Isolationsbereichs eine Membranstruktur (SSM) mit einer Fläche von etwa A ≈ 0,1 - 50 mm² und mit einer spezifischen elektrischen Leitfähigkeit von etwa Gₘ ≈ 1 - 100 nS/cm² und/oder mit einer spezifischen Kapazität von etwa Cₘ ≈ 10 - 1000 nF/cm² aufweist.

Die hohe spezifische Kapazität Cₘ, ist von besonderem Vorteil im Hinblick auf eine durchzuführende amperometrische Wirkstofftestung, bei welcher initiierte elektrische Aktionen der im Wesentlichen biologischen Einheiten als elektrische Ströme, nämlich als Verschiebungsströme oder kapazitive Ströme gemessen werden.

Im Hinblick auf das Signalrauschverhältnis ist ein entsprechender Abdichtwiderstand im Bereich von wenigen Nanosiemens von besonderem Vorteil.

Dieser kann gemäß einer anderen Ausführungsform der erfindungsgemäßen Sensorelektrodeneinrichtung auch durch Aufbringen einer Teflonschicht, zum Beispiel direkt auf die Metallelektrode, erreicht werden. Ein derartiges Vorgehen ist zum Beispiel für potentiometrische Wirkstofftestungen vollständig ausreichend, da es hier nicht auf eine hohe elektrische Kapazität ankommt, sondern wegen der Spannungsmessung auf den hohen Abdichtwiderstand.

Besonders einfache geometrische Verhältnisse ergeben sich, insbesondere im Hinblick auf die Reproduzierbarkeit der Messergebnisse, wenn der Träger, die Elektrode und/oder der Isolationsbereich und/oder deren Ober- oder Grenzflächenbereiche zumindest teilweise im Wesentlichen planar ausgebildet sind, insbesondere auch auf mikroskopischer Ebene oder Skala. Die Planarität gewährleistet, dass bestimmte Feldstärkeeffekte an Kanten oder Spitzen, die zum Durchbruch des Abdichtwiderstands führen könnten, ausbleiben. Des Weiteren ergibt sich im Hinblick auf den Austausch des vorzusehenden Messmediums im Betrieb der Vorteil einer homogenen Grenzflächenverteilung. Etwaige Protuberanzen oder Kavitäten würden an der Grenzfläche zwischen dem Isolationsbereich und dem Messmedium zu Konzentrationsinhomogenitäten führen, die sich unter Umständen nachteilig auf erreichte Nachweis- oder Messergebnisse auswirken könnten. Die Planarität, insbesondere der metallischen Grenzflächen, kann durch entsprechende Herstellungsverfahren, zum Beispiel durch epitaktisches Aufwachsen, Annealen oder dergleichen gewährleistet werden.

Zur externen Kontaktierung der Sensorelektrodeneinrichtung, zum Beispiel mit einem äußeren Messkreis oder dergleichen, ist ein Kontaktbereich vorgesehen, wobei insbesondere eine entsprechende Isolation zur Vermeidung sonstiger Kurzschlüsse, insbesondere in Bezug auf das Messmedium, ausgebildet ist.

Besonders vorteilhaft im Hinblick auf einen hohen Durchsatz bei entsprechend durchzuführenden Wirkort- und/oder Wirkstofftests ist es, wenn die erfindungsgemäße Sensorelektrodeneinrichtung so ausgebildet ist, dass sie, zumindest im Betrieb, gegenüber Flüssigkeitsströmungen mit hohen Strömungsgeschwindigkeiten, vorzugsweise im Bereich von etwa v ≈ 0,1 - 2 m/s im Wesentlichen konstante mechanische, elektrische und/oder strukturelle Eigenschaften, insbesondere im Bereich der Membranstruktur und/oder insbesondere im Hinblick auf die Anlagerung und/oder Anordnung von Primärträgern, aufweist. Diese geforderte und vorteilhafte Konstanz der mechanischen, elektrischen und/oder strukturellen Eigenschaften der erfindungsgemäßen Sensorelektrodeneinrichtung und insbesondere der darin vorgesehenen Membranstruktur ergibt sich bereits inhärent aus den zuvor genannten Maßnahmen zur Ausbildung der Elektrode und der die Elektrode abdeckenden Isolationsschichten, insbesondere in Form von Self-Assembling-Monoschichten aus einem Alkanthiol auf Gold mit einer entsprechenden Monoschicht aus Lipid in einem wässrigen Medium.

Vorteilhafterweise wird die erfindungsgemäße Sensoranordnung mit der beschriebenen Sensoreinrichtung in einem Verfahren zur amperometrischen und/oder potentiometrischen, pharmakologischen Wirkort- und/oder Wirkstofftestung und in einer Vorrichtung zur Durchführung eines solchen Verfahrens verwendet.

Bei der erfindungsgemäßen Sensoranordnung werden als Primärträger jeweils eine eukariontische Zelle, eine prokariontische Zelle, Organellen davon, eine bakterielle Einheit, eine virale Einheit und/oder dergleichen und/oder Bestandteile, Fragmente, insbesondere Membranfragmente oder dergleichen, und/oder Verbände davon in im Wesentlichen nativer und/oder in abgewandelter, insbesondere gereinigter, mikrobiologisch und/oder molekularbiologisch geänderter, Form vorgesehen.

Es ist somit grundsätzlich denkbar, dass isolierte und ganze Zellen als Primärträger entsprechender biologischer Einheiten, welche zu einer elektrischen Aktion aktivierbar sind, verwendet werden, seien diese pflanzlicher oder tierischer Herkunft. So ist zum Beispiel eine Untersuchung ganzer Herzzellen möglich und denkbar. Andererseits kann auch an die Untersuchung pflanzlicher Zellen, zum Beispiel von Algenzellen oder anderer Einzeller gedacht werden. Darüber hinaus können auch bestimmte Bakterien oder Viren als Ganzes untersucht werden. Ferner ist denkbar, durch bestimmte mikrobiologische oder biochemische Maßnahmen Bestandteile oder Fragmente von Zellen, Bakterien oder Viren, als Primärträger zu verwenden. Weiterhin ist auch denkbar, Verbände von Zellen, Bakterien oder dergleichen als Primärträger zu verwenden und diese an die entsprechende Sensorelektrodeneinrichtung zur Ausbildung einer erfindungsgemäßen Sensoranordnung anzukoppeln.

Des Weiteren besteht erfindungsgemäß die Möglichkeit, sämtliche dieser vorgeschlagenen Primärträger in ihrer nativen Form oder in einer abgewandelten Form zu verwenden. Dabei können zum Beispiel eukariontische Zellen, prokariontische Zellen oder Bakterien verwendet werden, die durch entsprechende Reinigungs-, mikrobiologische und/oder molekularbiologische Verfahren abgeändert wurden, zum Beispiel um bestimmte Proteine mit bestimmten gewünschten Eigenschaften bevorzugt auszubilden.

Neben den in natürlicher Form bereits bereitstehenden Primärträgern in Form von Zellen, Bakterien und dergleichen ist es auch denkbar, künstliche Primärträger zu erzeugen, zum Beispiel in Form von Vesikeln, Liposomen, mizellären Strukturen und/oder dergleichen. Diese werden dann gegebenenfalls mit entsprechenden biologischen Einheiten, welche zu einer elektrischen Aktion aktivierbar sind, versehen und/oder angereichert. Entsprechende Verfahren zur Rekonstitution von Membranproteinen oder dergleichen in Vesikeln oder Liposomen sind bekannt und können hier in vorteilhafter Art und Weise ausgenutzt werden, um besonders vorteilhafte Ausführungsformen der erfindungsgemäßen Sensoranordnung zu schaffen.

Als im Wesentlichen biologische Einheiten kommen sämtliche Einheiten in Frage, die zu einer zumindest teilweise elektrisch ausgebildeten Aktion veranlassbar sind. Insbesondere sind derartige biologische Einheiten denkbar, die zumindest zu einem teilweise elektrogenen und/oder elektrophoretischen Ladungsträgertransport und/oder zu einer zumindest teilweise elektrogenen oder elektrophoretischen Ladungsträgerbewegung aktivierbar sind und welche biologische, chemische und biochemische Einheiten darstellen. Dabei handelt es sich insbesondere um Transporteinheiten, die auf ihre Aktivierung hin Ladungsträger bewegen. Denkbar sind auch Bestandteile, Fragmente und/oder Verbände derartiger Einheiten, insbesondere Transporteinheiten.

Es bieten sich insbesondere als biologische Einheiten Membranproteine an, insbesondere Ionenpumpen, Ionenkanäle, Transporter, Rezeptoren und/oder dergleichen. In Bezug auf viele dieser biologischen Einheiten bestehen Erkenntnisse und/oder Vermutungen dahingehend, dass bestimmte Prozesse mit zumindest einem elektrogenen Teilschritt verbunden sind. Diese elektrogenen Teilschritte können mit einem tatsächlichen Stofftransport verbunden sein, wie zum Beispiel bei einem Kanal, einer Ionenpumpe, oder bestimmten Transportern. Es sind aber auch biologische Einheiten, insbesondere Membranproteine, bekannt, deren elektrische Aktivität nicht mit einem Nettostofftransport zusammenhängt, sondern lediglich mit einer, gegebenenfalls reversiblen, Ladungsverschiebung im Rahmen einer Konformationsänderung oder Bindung oder dergleichen. Auch solche elektrischen Aktivitäten sind grundsätzlich als kurzzeitige Verschiebungsströme und/oder Potenzialänderungen erfindungsgemäß messbar.

Die biologischen Einheiten, insbesondere die Membranproteine, können jeweils in im Wesentlichen nativer Form und/oder in abgewandelter, insbesondere gereinigter, mikrobiologisch und/oder molekularbiologisch geänderter Form vorgesehen sein. Zum einen können dadurch bestimmte native Eigenschaften zum Beispiel im Organismus vorhandener Proteine getestet und pharmakologisch untersucht werden. Andererseits bieten sich auch molekularbiologische oder gentechnisch initiierte Veränderungen an, die bestimmten Aspekte, zum Beispiel des Transports oder der pharmakologischen Wirkungsweise eines Wirkstoffes zu analysieren.

Besonders vorteilhaft ist es, dass Primärträger eines jeweils im Wesentlichen einheitlichen Typs von Primärträgern vorgesehen sind. Dies ist im Hinblick auf eine möglichst eindeutige Aussage und Analyse eines Wirkstofftests von Bedeutung und bezieht sich auf die geometrischen, physikalischen, chemischen, biologischen und molekularbiologischen Eigenschaften der Primärträger.

Das Nämliche gilt auch für die in dem Primärträger vorgesehenen biologischen Einheiten, insbesondere für die Membranproteine oder dergleichen. Hier sind biologische Einheiten eines jeweils im Wesentlichen einheitlichen Typs vorgesehen, insbesondere im Hinblick auf ihre geometrischen, physikalischen, chemischen, biologischen und molekularbiologischen Eigenschaften. Zusätzlich sollen die biologischen Einheiten in vorteilhafter Weise im Hinblick auf ihre Orientierung und/oder im Hinblick auf ihre Aktivierbarkeit in Bezug auf den jeweiligen Primärträger in etwa einheitlich sein.

Zur Erzielung einer möglichst hohen Signalqualität ist es von Vorteil, dass die Oberflächen der Primärträger und/oder der Sekundärträger so ausgebildet sind, dass eine Anlagerung und/oder Anordnung der Primärträger am Sekundärträger begünstigt wird. Dadurch erhält man zum einen eine besonders hohe Anzahl angelagerter Primärträger und/oder einen besonders innigen Kontakt der Primärträger am Sekundärträger, wodurch die elektrische Kopplung und somit das Signal-zu-Rauschverhältnis gesteigert werden.

Die Anlagerung kann z.B. über die sogenannte Lipid-Lipid-Wechselwirkung zwischen Primärträger, z.B. Vesikel, und dem Sekundärträger, z.B. Lipid-Thiol-SSM, gesteuert sein. Andrereseits ist auch eine kovalente Bindung der Primärträger an der Oberfläche der Sekundärträger denkbar, z.B. in Form eines Biotin-Streptavidin-Schemas oder im Sinne einer His-Tag-Kopplung.

Dabei ist es von besonderem Vorteil, wenn die Oberflächen der Primärträger und der Sekundärträger entgegengesetzt polar zueinander ausgebildet sind. Dies fördert die Anlagerungsrate der Primärträger am Sekundärträger sowie die Stärke des Kontakts zwischen diesen.

Von besonderem Vorteil ist es, wenn als Primärträger im Wesentlichen gleich wirkende und/oder gleichartige Vesikel oder Liposomen, vorzugsweise aus einem Lipid, vorgesehen sind, in und/oder an deren Membran Einheiten im Wesentlichen eines Typs von Membranproteinen in vorzugsweise im Wesentlichen orientierter Form ein- und/oder angelagert sind.

Die erfindungsgemäße Sensoranordnung wird vorteilhafterweise von einem Verfahren zur amperometrischen und/oder potentiometrischen, insbesondere pharmakologischen Wirkort- und/oder Wirkstofftestung und/oder bei einer Vorrichtung zur Durchführung eines solchen Verfahrens verwendet.

Die erfindungsgemäße Vorrichtung für die amperometrische und/ oder potentiometrische, pharmakologische Wirkort- und/oder Wirkstofftestung weist mindestens einen Messbereich auf. Des Weiteren ist als Messsonde zur Aufnahme und/oder zur Ausbildung von Messsignalen mindestens eine erfindungsgemäße Sensoranordnung vorgesehen. Des Weiteren ist eine Datenerfassungs-/Steuereinrichtung vorgesehen, welche zumindest zur Erfassung von Messsignalen der jeweiligen Messsonde ausgebildet ist. Essentiell ist das Vorsehen einer Austausch-/Mischeinrichtung, welche zum Verfügungstellen, Austausch, Mischen und/oder Einstellen des Messmediums ausgebildet ist. Die Sensoranordnung ist in Messbereich als Messraum oder Messort angeordnet.

Eine derartige Vorrichtung kann zum Beispiel in Form einer sogenannten Patchclamp-Apparatur aufgebaut sein, bei welcher ein Elektrolytbad den eigentlichen Messbereich darstellt. Als Messsonde wäre dabei herkömmlicherweise die sogenannte Patchelektrode oder Patchpipette vorgesehen, an welcher eine zu untersuchende biologische Einheit, zum Beispiel eine Zelle oder dergleichen, angesaugt und gehaltert wird. Erfindungsgemäß ist hier die oben beschriebene Sensoranordnung ausgebildet. Die Datenerfassungs-/Steuereinrichtung wird von der gesamten Messelektronik, entsprechenden Mikromanipulatoren und von der Steuerelektronik zum Aufbau und Mischen des Elektrolytbades gebildet. Die Austausch-/Mischeinrichtung kann zum Beispiel aus entsprechenden Schlauchsystemen mit daran angeschlossenen Perfusoren oder dergleichen bestehen.

Die erfindungsgemäße Vorrichtung für die amperometrische und/ oder potentiometrische, pharmakologische Wirkort- und/oder Wirkstofftestung weist also als Messsonde jeweils die oben beschriebene erfindungsgemäße Sensoranordnung auf. Dadurch ergibt sich in vorteilhafter Weise eine besonders stabile und robuste Messanordnung, mit welcher für eine gegenüber der herkömmlichen Vorgehensweise erheblich verlängerte Zeitspanne eine Vielzahl von Testläufen unter den verschiedensten Versuchsbedingungen durchgeführt werden kann, und zwar ohne Einbußen in der Nachweisgenauigkeit, der Signalqualität oder sonstiger Eigenschaften der Messsonde. Des Weiteren kann aufgrund der Robustheit der Messsonde mit höherer Geschwindigkeit gearbeitet werden, dies betrifft sowohl die Handhabung des eigentlichen Sensors als auch die Austauschgeschwindigkeit oder Strömungsgeschwindigkeit des fluiden Messmediums im Messbereich. Herkömmliche Patchclamp- und/oder Voltageclamp-Anordnungen sind im Gegensatz dazu bekannterweise im Hinblick auf die Reproduzierbarkeit der Messergebnisse und der Stabilität des Messsystems problematisch.

Vorteilhafterweise sind, insbesondere über die Austauscheinrichtung, über das Messmedium die Messbedingungen, insbesondere die pharmakologischen Bedingungen, gemäß einer weiteren Ausführungsform der erfindungsgemäßen Vorrichtung für die pharmakologische Wirkstofftestung definiert einstellbar und/oder änderbar, insbesondere in kontinuierlicher Art und Weise, vorzugsweise nach Art eines kontinuierlichen Fließsystems.

Im Gegensatz zum Stand der Technik, bei welchem eine derartig wohldefinierte Einstellbarkeit und/oder Änderbarkeit der Messbedingungen nur schwer oder unter erhöhtem Aufwand möglich ist, ist aufgrund der Stabilität der verwendeten erfindungsgemäßen Messsonde ein Austausch und/oder eine Änderung des Messmediums leicht möglich. Durch den Austausch können auch entsprechende Änderungen im Hinblick auf die Substratbedingungen oder sonstige Eigenschaften der Messumgebung auf einfache Art und Weise und in kürzerer Zeit erreicht werden.
Insbesondere bietet es sich an, als Austausch-/Mischeinrich-tung ein Pumpensystem, Perfusorsystem und/oder dergleichen zu verwenden, um eine Messanordnung zu schaffen, bei welcher die Messsonde im Rahmen eines Fließsystems ständig vom Messmedium umströmt wird. In einem derartigen Fließsystem können dann durch externes Zumischen entsprechende zu untersuchende Messbedingungen geschaffen werden.

Dabei ist es insbesondere von Vorteil, dass im Messbereich Fließgeschwindigkeiten oder Strömungsgeschwindigkeiten von etwa v ≈ 0,1 - 2 m/s erzeugbar sind, insbesondere gerade im Bereich, Nahbereich oder der Nachbarschaft der Messsonde und/oder insbesondere durch die vorgesehene Austausch-/Mischeinrichtung.

Der Vorteil der hohen Fließ- und/oder Strömungsgeschwindigkeiten ergibt sich zum einen aufgrund der mechanischen Stabilität der erfindungsgemäßen Sensoranordnung und der ihr zugrundeliegenden Sensorelektrodeneinrichtung.

Darüber hinaus ist es aber besonders vorteilhaft, dass neben der Verwendung von ganzen Zellen, Bakterien oder dergleichen, welche vergleichsweise groß ausgebildet sind, in vorteilhafter Weise auch Vesikel und Liposomen sowie Membranfragmente als Primärträger für die biologischen Einheiten, insbesondere Membranproteine, eingesetzt werden können. Vesikel, Liposomen, Membranfragmente und dergleichen sind vergleichsweise klein ausgebildet und besitzen im Verhältnis zu ihrer Größe oder ihrer Oberfläche eine vergleichsweise stärkere Neigung zur Anlagerung oder Adsorption an der Oberfläche der Messsonde. Darüber hinaus erfahren sie aufgrund ihrer geringeren Oberfläche im Bereich der Strömung des Messmediums im Vergleich zu ganzen Zellen oder dergleichen sehr viel geringere Scherkräfte, so dass sie auch bei höheren Fließ- und/oder Strömungsgeschwindigkeiten an der erfindungsgemäßen Sensorelektrodeneinrichtung im Rahmen der erfindungsgemäßen Sensoranordnung haften bleiben, so dass sich die Versuchsbedingungen oder Testbedingungen nicht ändern.

Vorteilhafterweise ist der Messbereich einfach als im Wesentlichen geschlossenes Gefäß oder als Gefäßeinrichtung ausgebildet. Dabei ist es gemäß einer weiteren Ausführungsform der erfindungsgemäßen Vorrichtung vorgesehen, entsprechende Zuführ- und/oder Abführeinrichtungen zur strömungsmäßigen Verbindung mit der Austausch-/Mischeinrichtung auszubilden. Der Messbereich kann zum Beispiel als eine Art Kompartment oder Küvette ausgebildet sein, an deren Bodenbereich die Messsonde in Form einer erfindungsgemäß ausgebildeten Sensoranordnung angeordnet ist.

Bei einer weiteren vorteilhaften Ausführungsform der erfindungsgemäßen Vorrichtung für die amperometrische und/ oder potentiometrische, pharmakologische Wirkort- und/oder Wirkstofftestung ist eine Mehrzahl integrierter Sensoranordnungen, vorzugsweise in separaten, strömungsmäßig und elektrisch voneinander entkoppelten und unabhängigen Vertiefungsbereichen einer Mikroplatte oder Mikrotitaplatte vorgesehen, vorzugsweise um 8, 12, 96 Messkanäle auf einem Raster auszubilden.

Es ist also denkbar, dass eine Mehrzahl von Messsonden vorgesehen ist, insbesondere um einen Parallelbetrieb für mehrere unabhängige Testreihen simultan durchzuführen. Dabei ist es dann ferner vorteilhaft, dass die Datenerfassungs-/Steuereinrichtung ausgebildet ist, Messsignale der Mehrzahl von Messsonden voneinander unabhängig und/oder entkoppelt aufzunehmen und/oder zu erfassen. Dies gewährleistet, dass die unterschiedlichen Messsonden und deren Messsignale voneinander separiert ausgewertet und einer genaueren Betrachtung unterzogen werden können und ermöglicht somit die Realisierung voneinander unabhängiger und simultan durchgeführter Experimente oder Testvorgänge, wobei insbesondere ein höherer Testdurchsatz mit geringeren Kosten pro Messung erzielbar ist.

Darüber hinaus ist es dann von Vorteil, dass die Austausch-/Mischeinrichtung und/oder der Messbereich ausgebildet sind, für die Mehrzahl von Messsonden unabhängig und/oder entkoppelt voneinander entsprechende Messbedingungen auf definierte Art und Weise einzustellen und/oder zu ändern. Es kann sich bei dem Messbereich zum Beispiel um eine Anordnung voneinander strömungsmäßig getrennter Küvetten oder Kompartments handeln. Dabei kann auch eine gemeinsame Austausch-/Messeinrichtung vorgesehen sein, über welche für alle Messbereiche simultan dieselben Messbedingungen, zum Beispiel hinsichtlich des Messmediums oder dergleichen, geschaffen werden. Wichtig dabei ist aber die strömungsmäßige und vor allem elektrische Entkopplung der voneinander getrennt zu betrachtenden Messbereiche.

Insbesondere ist es aber von Vorteil, eine Messanordnung für die erfindungsgemäße Vorrichtung zu schaffen, bei welcher die Testvorgänge an Messsensoren in mehr oder weniger miniaturisierter Form durchgeführt werden können. Zum Beispiel kann der Messbereich dazu in Form oder im Raster einer Mikroplatte oder Mikrotiterplatte oder dergleichen ausgebildet sein, wobei eine Mehrzahl integrierter Messsonden, vorzugsweise in separaten und strömungsmäßig und/oder elektrisch voneinander entkoppelten, und/oder voneinander unabhängigen Vertiefungsbereichen davon ausgebildet ist. Vorzugsweise können die Vertiefungsbereiche und die darin vorgesehenen Messsonden in Form eines Rasters auf einer derartigen Mikroplatte angeordnet sein, um eine Anordnung von 4, 8, 12, 96 oder dergleichen parallelen Messkanälen zu realisieren. Dabei können dann entsprechend gängige 4-, 8- 12-, 96-kanalige Pipettierautomaten für die Probenzugabe zum Einsatz kommen.

Wie oben bereits erläutert wurde, wird bei dem erfindungsgemäßen Verfahren zur amperometrischen und/oder potentiometrischen, pharmakologischen Wirkort- und/oder Wirkstofftestung eine zu verwendende oder zu untersuchende biologische Einheit auf oder in mindestens einer Messsonde vorgesehen. Die Messsonde ihrerseits wird jeweils in einem Messmedium angeordnet, wobei das Messmedium zum Beispiel ein im Wesentlichen wässriges Medium sein kann. Des Weiteren wird beim eigentlichen Testvorgang über die vorgesehene Messsonde die jeweils initiierte elektrische Aktion der im Wesentlichen biologischen Einheit gemessen, insbesondere als elektrische Spannung und/oder als elektrischer Strom.

Die verfahrensmäßige erfindungsgemäße Lösung der zugrundeliegenden Aufgabe besteht nun darin, dass man bei dem an sich bekannten Verfahren gerade die oben im Detail beschriebene Sensorelektrodeneinrichtung, die entsprechende Sensoranordnung und/oder die beschriebene erfindungsgemäße Vorrichtung zur amperometrischen und/oder potentiometrischen, insbesondere pharmakologischen Wirkort- und/oder Wirkstofftestung heranzieht.

Dabei ist es insbesondere vorteilhaft, dass die Messsonde jeweils von dem vorgesehenen Messmedium zumindest teilweise und/oder zumindest zeitweise umströmt oder angeströmt wird. Durch dieses Vorgehen ist ein leichter Wechsel der Testbedingungen, nämlich durch einen externen Misch- oder Austauschvorgang, auf störungsfreie Art und Weise erreichbar, im Gegensatz zur Vorgehensweise beim Stand der Technik, zum Beispiel bei den Messverfahren gemäß der Patchclamp-Technik oder Voltageclamp-Technik.

Besonders vorteilhaft ist das erfindungsgemäße Verfahren dadurch, dass eine Strömungsgeschwindigkeit und/oder Fließgeschwindigkeit des Messmediums von etwa v ≈ 0,1 - 2 m/s verwendet wird, weil sich auf der Grundlage dieser hohen Strömungsgeschwindigkeiten auch gerade kinetische Untersuchungen an den biologischen Einheiten, insbesondere an den Membranproteinen, durchführen lassen, bei welchen mit zeitlich hoher Auflösung die Signalentwicklung aufgenommen und charakterisiert und auf die einzelnen Funktionsschritte der biologischen Einheit zurückgeführt werden.

Aufgrund der Stabilität der bei der erfindungsgemäßen Verfahren verwendeten Sensorelektrodeneinrichtung, Sensoranordnung und/oder -vorrichtung ergibt sich in vorteilhafter Weise die Möglichkeit, aufeinanderfolgend eine Mehrzahl oder Vielzahl von Tests durchzuführen, insbesondere durch ein aufeinanderfolgendes Austauschen und/oder Einstellen oder Mischen des Messmediums, wobei gegebenenfalls zwischengeschaltet Wasch- oder Spülvorgänge vorgesehen sein können. Durch dieses Vorgehen kann bei ein und derselben biologischen Einheit, zum Beispiel bei einem gegebenen Rezeptor oder einem anderen Membranprotein, welches, einmal in einem Vesikelsystem inkorporiert, an einer erfindungsgemäß im Verfahren vorgesehenen Sensorelektrodeneinrichtung eingelagert wurde, eine Vielzahl von Substanzen, welche in einem Mischvorgang dem Messmedium zugefügt werden, getestet werden. Geschieht dies gleichzeitig auch im Parallelbetrieb, so kann in einem Messdurchgang eine Vielzahl im Wesentlichen gleicher Tests durchgeführt werden, um zum Beispiel eine entsprechende statistische Evaluierung der Wirkungsweise bestimmter Wirkstoffe auf eine gegebene biologische Einheit zu erhalten.

Oft ist nicht so sehr die Wirkweise eines bestimmten Wirkstoffes an einer bekannten biologischen Einheit von Interesse, sondern vielmehr soll die Funktionsweise einer diesbezüglich unbekannten biologischen Einheit aufgeklärt werden. Dabei ist naturgemäß eine Vielzahl von einander unabhängiger Tests durchzuführen.

Dies ist insbesondere beim sogenannten Deorphaning der Fall. Dabei wird von einem Orphan ausgegangen. Dieses ist ein genetische Informationseinheit, durch welche eine bestimmte, aber bisher funktionsunbekannte biologische Einheit, z.B. ein Membranprotein oder dergleichen, kodiert wird.

Entsprechend ist es gemäß einer besonders bevorzugten Ausführungsform des erfindungsgemäßen Verfahrens vorgesehen, dass ein Deorphaningprozess mit einer Mehrzahl oder eine Vielzahl von Tests durchgeführt wird, wobei als biologische Einheit ein Produkt oder dergleichen eines - insbesondere funktionsunbekannten - Orphans oder dergleichen verwendet wird, insbesondere um dessen Funktionalität aufzuklären. Entsprechend ist es erfindungsmäß vorgesehen, die erfindungsgemäße Sensorelektrodeneinrichtung, die erfindungsgemäße Sensoranordnung und/oder die erfindungsgemäße Vorrichtung bei einem Deorphaningvorgang oder - prozess zu verwenden.

Nachfolgend wird die vorliegende Erfindung anhand einer schematischen Zeichnung auf der Grundlage bevorzugter Ausführungsbeispiele näher erläutert.
- **Fig. 1**: zeigt eine schematische und teilweise geschnittene Seitenansicht eines Ausführungsbeispiels der erfin- dungsgemäßen Vorrichtung unter Verwendung der erfin- dungsgemäßen Sensoranordnung.
- **Fig. 2**: zeigt eine Ausführungsform einer erfindungsgemäßen Sensoranordnung mit einem Membranfragment als Primär- träger.
- **Fig. 3A-D**: zeigen in einer schematischen Seitenansicht eine Aus- führungsform des erfindungsgemäßen Verfahrens zur pharmakologischen Wirkort- und/oder Wirkstofftestung.
- **Fig. 4A-C**: zeigen in einer schematischen Seitenansicht eine an- dere Ausführungsform des erfindungsgemäßen Verfahrens zur pharmakologischen Wirkort- und/oder Wirkstofftes- tung.

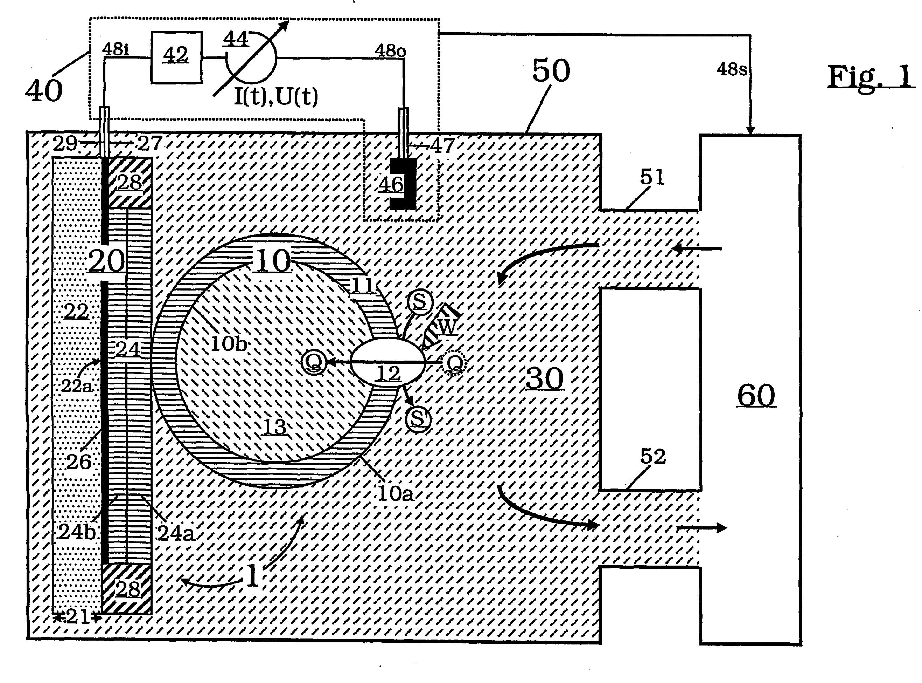

Fig. 1 zeigt in einer schematischen und teilweise geschnittenen Seitenansicht eine Ausführungsform der erfindungsgemäßen Vorrichtung zur amperometrischen und/oder potentiometrischen pharmakologischen Wirkstofftestung.

Ein Messbereich 50 in Form eines im Wesentlichen geschlossenen Gefässes bildet zusammen mit einer Austausch-/Mischeinrichtung 60, zum Beispiel in Form eines Perfusorsystems oder einer Pumpenanlage, einen geschlossenen Flüssigkeitskreislauf. Die Kommunikation der als Messmedium 30 dienenden Flüssigkeit erfolgt über entsprechende Zuführ- und Abführeinrichtungen 51 bzw. 52. Das Messmedium 30 kann dabei eine wässrigere Elektrolytlösung sein, die bestimmte Ionenanteile, eine gegebene Temperatur, einen bestimmten pH-Wert usw. aufweist. Des Weiteren sind im Messmedium 30 gegebenenfalls bestimmte Substratstoffe S und/oder bestimmte Wirkstoffe W enthalten, oder sie werden in späteren Verfahrensschritten durch die Austausch-/Mischeinrichtung 60 hinzugefügt.

Im Messbereich 50 ist eine erfindungsgemäße Sensoranordnung 1 vorgesehen. Die Sensoranordnung 1 besteht aus Primärträgern 10, welche am Oberflächenbereich 24a der als Sekundärträger dienenden Sensorelektrodeneinrichtung 20 angelagert sind.

In dem in Fig. 1 in schematischer und nicht maßstabsgetreuer Form gezeigten Ausführungsbeispiel ist nur ein einziger Primärträger 10 gezeigt. Dieser besteht aus einem Lipidvesikel oder Liposom in Form einer im Wesentlichen hohlkugelförmig und geschlossen ausgebildeten Lipiddoppelschicht oder Lipidmembran 11. In diese Lipiddoppelschicht 11 des als Primärträger 10 dienenden Vesikels ist als im Wesentlichen biologische Einheit 12 ein Membranprotein membrandurchgreifend eingelagert.

Durch Umsetzung eines im Messmedium 30 vorhandenen Substrats S zu einem umgesetzten Substrat S' werden im Membranprotein 12 bestimmte Prozesse initiiert, die in dem in Fig. 1 gezeigten Fall zu einem Stofftransport einer Spezies Q von der extravesikulären Seite oder Außenseite 10a des Vesikels 10 zur intravesikulären Seite oder Innenseite 10b des Vesikels 10 führt. Ist die Spezies Q mit einer elektrischen Ladung behaftet, so führt der Transport dieser Spezies Q von der Seite 10a zur Seite 10b zu einem Nettoladungstransport, welcher mit einem elektrischen Strom von der Außenseite 10a des Vesikels 10 zur Innenseite 10b des Vesikels 10 korrespondiert.

Zum einen sind in jedem Vesikel 10 in der Regel eine Vielzahl im Wesentlichen identischer Membranproteinmoleküle 12 in im Wesentlichen gleicher Orientierung in der Membran 11 des Vesikels 10 eingebaut. Werden diese im Wesentlichen simultan aktiviert - z.B. durch einen durch Mischen initiierten Konzentrationssprung in der Konzentrations des Substrats S von einem nicht aktivierenden Messmedium N, 30 ohne Substrat S zu einem aktivierenden Messmedium A, 30 mit Substrat S - so führt das zu einem messbaren elektrischen Strom.

Dieser Ladungsträgertransport ist deshalb messbar, weil eine Vielzahl von Primärträgern 10 oder Vesikeln an der Oberfläche 24a der Sensorelektrodeneinrichtung 20 angelagert sind, so dass sich bei Aktivierung einer Vielzahl von Proteinmolekülen 12 in einer Vielzahl von Vesikeln vor der Oberfläche 24a der Sensorelektrodeneinrichtung 20 eine Raumladung bestimmter Polarität ausbildet. Diese Raumladung wirkt dann auf die Elektrode 26, die in dem in Fig. 1 gezeigten Fall auf einem Träger 22 aus Glas in Form einer Goldschicht aufgedampft ist und durch eine als Isolationsbereich 24 dienende Doppelschicht aus einer unteren Schicht 24b und einer als Oberfläche dienenden Oberschicht 24a abgedeckt und gegenüber dem Messmedium 30 elektrisch isoliert wird.

Die Oberfläche oder obere Schicht 24a des Isolationsbereichs 24 ist zum Beispiel ein zur Lipiddoppelschicht 11 des Vesikels 10 kompatible Lipidmonoschicht, die mittels eines Self-Assemb-ling-Vorgangs auf einer die untere Schicht 24b bildenden Alkanthiolmonoschicht ausgebildet ist, so dass die Abfolge der Schichten 24b und 24a, nämlich die Abfolge aus einer Alkanthiolmonoschicht und einer Lipidmonoschicht auf einem festkörperartig ausgebildeten Goldsubstrat als Elektrode 26 eine Membranstruktur SSM bildet, die auch als festkörperunterstützte Membran bezeichnet wird (SSM: Solid Supported Membrane).

Über eine Anschlussleitung 48i ist die Sensoranordnung 1 und insbesondere die Sensorelektrodeneinrichtung 20 mit einer Datenerfassungs-/Steuereinrichtung 40 verbunden. Diese weist einen Messeinrichtung 44 auf, in welchem in zeitlicher Abhängigkeit ein elektrischer Strom I(t) oder eine elektrische Spannung U(t) gemessen werden kann. Des Weiteren ist eine Verstärkereinrichtung 42 vorgesehen, in welcher die Messsignale gefiltert und/oder verstärkt werden. Über eine Steuerleitung 48s wird die Wirkstofftestung durch Steuerung der Austausch-/Mischeinrichtung 60 geregelt. Über eine weitere Leitung 48o wird der elektrische Stromkreis mittels einer Gegenelektrode 46, zum Beispiel in Form einer Pt/Pt-Elektrode oder mittels einer Ag/AgCl-Elektrode geschlossen. Isolationen 28, 27 und 47 verhindern Kurzschlüsse der SSM bzw. der Gegenelektrode 46 gegenüber dem Messmedium 30.

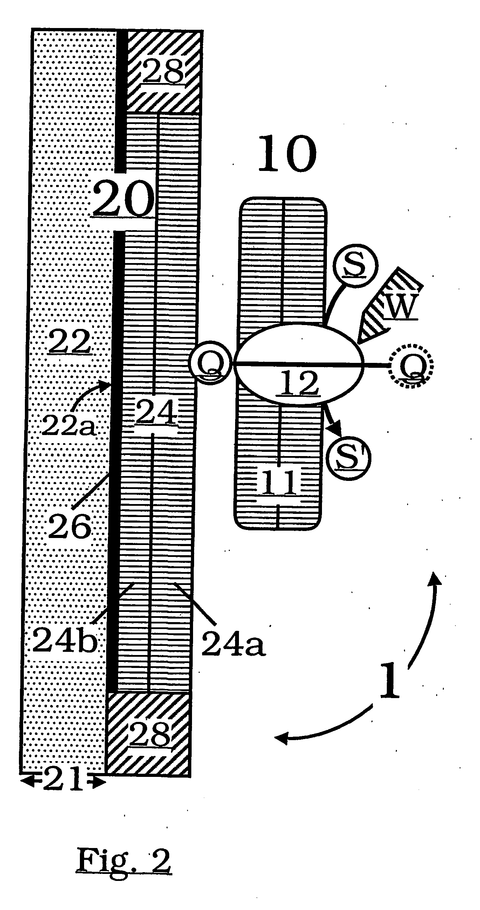

Fig. 2 zeigt in schematischer und teilweise geschnittener Seitenansicht eine Ausführungsform der erfindungsgemäßen Sensoranordnung 1, bei welcher als Primärträger 10 anstelle eines Vesikels oder Liposoms ein Membranfragment 10 vorgesehen ist, in welches in orientierter Art und Weise ein als biologische Einheit 12 dienendes Membranprotein eingelagert ist. Auch in Bezug auf die Ausführungsform der Fig. 2 ist festzuhalten, dass die Darstellung nicht maßstabsgetreu ist, und zum anderen in der Regel eine große Mehrzahl von Membranfragmenten gleichzeitig auf der SSM oder der Oberfläche 24a der als Sekundärträger dienenden Sensorelektrodeneinrichtung 20 angelagert oder adsorbiert sind.

Auch hier ist wieder gezeigt, dass durch Umsetzung des im Messmedium 30 vorgesehenen Substrats S zu einem umgesetzten Substrat S' ein Stofftransport der Spezies Q von einer Seite 10a des Membranfragments 10 zur gegenüberliegenden Seite 10b erfolgt, welcher über den entsprechenden Nettoladungstransport und den damit verbundenen Verschiebungsstrom in zeitlich abhängiger Form nachgewiesen werden kann.

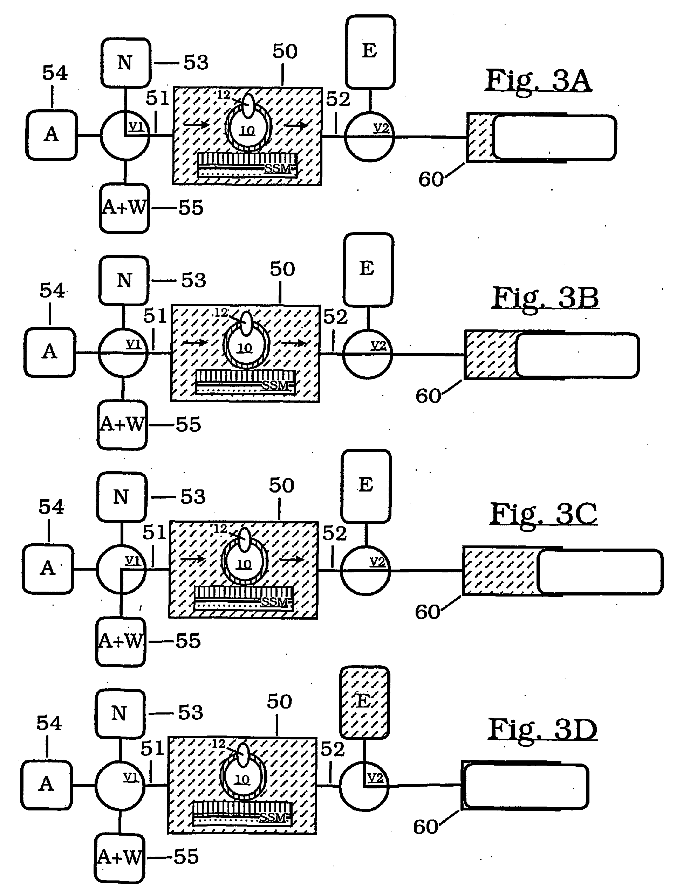

Die Fig. 3A bis 3D zeigen eine Ausführungsform des erfindungsgemäßen Verfahrens zur amperometrischen und/oder potentiometrischen, pharmakologischen Wirkort- und/oder Wirkstofftestung.

In einem als Küvette ausgebildeten Messbereich 50 ist an einer als Sensorelektrodeneinrichtung 20 dienenden SSM ein Assemble von Vesikeln 10 mit einem dort eingelagerten Membranprotein 12 adsorbiert. Die so ausgebildete Sensoranordnung 1 ist dabei im Messbereich 50 in einem vorgesehenen Messmedium 30 inkubiert.

Der Messbereich 50 ist über eine Zuführleitung 51 über eine vorgesehene erste Ventileinrichtung V1 steuerbar wahlweise mit einer Mehrzahl von Vorratsgefäßen 53, 54 und 55 verbunden, in welchen bestimmte Volumina des Messmediums 30 mit bestimmten weiteren Inhaltsstoffen versehen, vorgesehen sind. So enthält das erste Vorratsgefäß 53 der Fig. 3A bis 3D ein nicht aktivierendes Messmedium N, welches so gewählt ist, dass die in den Vesikeln 10 eingelagerten Membranproteine 12 durch die Zusammensetzung des Messmediums des Typs N nicht zu einer elektrischen Aktion initiiert werden. Im zweiten Vorratsgefäß 54 der Fig. 3A bis 3D ist ein Messmedium A enthalten, durch welches die Membranproteine 12 in den Vesikeln 10 zu einer elektrischen Aktion, also zu einem Ladungstransport oder dergleichen, aktivierbar sind. Im dritten Vorratsgefäß 55 der Fig. 3A bis 3D schließlich ist dem aktivierenden Messmedium A des Gefäßes 54 ein Wirkstoff W hinzugefügt worden, dessen Wirkung auf die Aktivität des Proteins 12 in den Vesikeln 10 ermittelt werden soll.

Über eine Abführeinrichtung 52 oder Abführleitung ist der Messbereich 50 oder die Küvette über eine zweite Ventileinrichtung V2 zumindest mit einem Teil der Austausch-/Mischein-richtung 60 verbunden. Des Weiteren kann über das Ventil V2 ein Entsorgungsgefäß E hinzugeschaltet werden, zum Beispiel um die Austausch-/Mischeinrichtung 60 zu entleeren.

In einer ersten Phase der pharmakologischen Wirkstofftestung, welche in Fig. 3A gezeigt ist, ist der Messbereich 50 über die erste Ventileinrichtung V1 und die Zuführeinrichtung 51 mit dem ersten Vorratsgefäß 53 verbunden. Andererseits ist der Messbereich 50 über die Abführeinrichtung 52 und über die zweite Ventileinrichtung V2 mit der Austausch-/Mischeinrichtung 60 verbunden. Durch Betrieb der Austausch-/Mischeinrichtung 60 wird eine Saugkraft über die miteinander verbundenen Bereiche ausgeübt, so dass aus dem Vorratsgefäß 53 das nicht aktivierende Messmedium N durch die Küvette des Messbereichs 50 strömt und die SSM sowie die proteinhaltigen Vesikel 10 umspült oder umströmt. In diesem Zustand kann keinerlei elektrische Aktivität des Proteins 12 gemessen werden, und diese Phase dient der Equilibration der SSM sowie der Aufnahme von Störsignalen und dem Abgleich des Rauschens.

In der in Fig. 3B gezeigten zweiten Testphase wird über die Datenerfassungs-/Steuereinrichtung 40 die erste Ventileinrichtung V1 so geschaltet, dass die Zuführeinrichtung 51 des Messbereichs 50 mit dem zweiten Vorratsgefäß 54 kommunizierend verbunden ist, so dass auf Betrieb der Austausch-/Mischein-richtung 60 hin das aktivierende Messmedium A durch die Küvette des Messbereichs 50 strömt, die SSM sowie die proteinhaltigen Vesikeln strömt und umspült und somit die in den Vesikeln 10 enthaltenen Membranproteine 12 zu einem elektrogenen Ladungstransport anregt, welcher dann über die in den Fig. 3A - 3D nicht gezeigte Datenerfassung nachgewiesen werden kann.

In einer in Fig. 3C gezeigten dritten Phase des Verfahrens der pharmakologischen Wirkstofftestung wird die erste Ventileinrichtung V1 derart geschaltet, dass der Messbereich 50 mit dem dritten Vorratsgefäß 55 strömungsmäßig verbunden ist, so dass auf Betrieb der Austausch-/Mischeinrichtung 60 hin nunmehr das aktivierende Messmedium A unter Zusatz des Wirkstoffes W durch die Küvette des Messbereiches 50 strömt und somit die SSM sowie die proteinhaltigen Vesikel 10 umspült.

Durch Aufnahme etwaiger elektrischer Signale unter dem Einfluss des hinzugefügten Wirkstoffes W kann im Vergleich zu den während der Phase der Fig. 3B aufgenommenen Signale der Einfluss des Wirkstoffes auf die Aktivität des Proteins 12 in den Vesikeln im Wesentlichen ermittelt werden.

In der Phase, welche in Fig. 3D gezeigt ist, wird die in der Austausch-/Mischeinrichtung 60 aufgenommene Flüssigkeitsmenge in das Entsorgungsgefäß E hin entsorgt, wobei durch die zweite Ventileinrichtung V2 die Austausch-/Mischeinrichtung 60 ausschließlich mit dem Entsorgungsgefäß E verbunden ist.

Selbstverständlich kann die in den Fig. 3A bis 3D gezeigte Anordnung bzw. das damit in Zusammenhang stehende Testverfahren auch komplexer ausfallen und weitere Messzwischenschritte, Spül- und Waschvorgänge enthalten.

Grundsätzliche Vorteile der vorliegenden Erfindung sind die hohe mechanische Stabilität der vorgesehenen Sensorelektrodeneinrichtung und der Sensoranordnung und damit einhergehend die große Einsatzbereitschaft, die einfache Handhabbarkeit und geringer Störunanfälligkeit. In der Verwendung der erfindungsgemäßen Sensorelektrodeneinrichtung und der erfindungsgemäßen Sensoranordnung ergeben sich im Rahmen von pharmakologischen Wirkstofftests eine lange Lebensdauer, eine hohe Zuverlässigkeit, eine geringe Störanfälligkeit sowie insbesondere ein gegenüber herkömmlichen Verfahren maßgeblich gesteigerter Testdurchsatz, wodurch entsprechende Testverfahren kostengünstig ausgearbeitet und durchgeführt werden können.

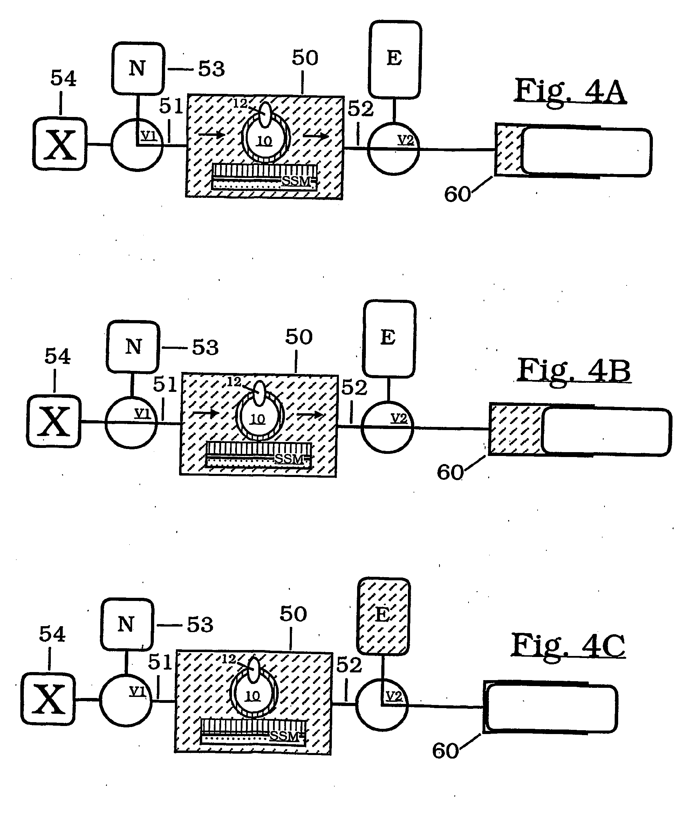

Die Fig. 4A bis 4C beschreiben eine andere Ausführungsform des erfindungsgemäßen Verfahrens. Vorgesehen sind dabei aber nur zwei Vorratsgefäße 53 und 54 vorgesehen sind. Das Vorratsgefäß 53 enthält wieder eine nich aktivierende Lösung N. Das Vorratsgefäß 54 enthält als X entweder in einem ersten Verfahrensschritt die aktivierende Lösung A und in einem zweiten Verfahrensschritt eine aktivierende Lösung A+W mit zu testendem Wirkstoff W. Im ersten Fall X=A wird die Aktivierung der biologischen Einheit als Referenzsignal gemessen. Im zweiten Fall X=A+W wird dann der Effekt des Wirkstoffes auf die Aktivierung messbar.

Im einfachsten Fall kann der Wirkstoff W identisch sein mit dem S und/oder mit der Transportspezies Q.

### Bezugszeichenliste

- 1: Sensoranordnung
- 10: Primärträger, Vesikel, Membranfragment
- 10a: Oberfläche, Außenseite, extravesikuläre Seite
- 10b: Innenseite, intravesikuläre Seite
- 11: Membran
- 12: biologische Einheit, Membranprotein
- 13: Innenmedium
- 20: Sensorelektrodeneinrichtung
- 21: Elektrodenbereich
- 22: Träger
- 22a: Oberflächenbereich
- 24: Isolationsbereich
- 24a: Oberschicht, Oberflächenbereich, Lipidmonoschicht
- 24b: Unterschicht, Thiol-/Mercaptanmonoschicht
- 26: Elektrode
- 27: Isolation
- 28: Isolation
- 29: Anschluss
- 30: Messmedium
- 40: Datenerfassungs-/Steuereinrichtung
- 42: Verstärkereinrichtung
- 44: Messeinrichtung
- 46: Gegenelektrode
- 48i,o: Anschlussleitungen
- 48s: Steuerleitung
- 50: Messbereich, Gefäß, Küvette
- 51: Zuführeinrichtung
- 52: Abführeinrichtung
- 53: Vorratsgefäß
- 54: Vorratsgefäß
- 55: Vorratsgefäß
- 60: Austausch-/Mischeinrichtung
- 100: Messvorrichtung

- A: aktivierendes Messmedium
- A+W: aktivierendes Messmedium mit Wirkstoff W
- Cₘ: spezifische Kapazität
- E: Entsorgung
- Gₘ: spezifische Leitfähigkeit
- N: nicht aktivierendes Messmedium
- I (t): Stromsignal
- Q: Ladungsträger
- S: Substrat
- S': umgesetztes Substrat
- SSM: Membranstruktur, festkörper unterstützte Membran
- U(t): Spannungssignal
- V: Fließgeschwindigkeit, Strömingsgeschwindigkeit
- V1: erste Ventileinrichtung
- V2: zweite Ventileinrichtung
- W: Wirkstoff

## Patentansprüche

1. Sensoranordnung für die amperometrische und/oder potentiometrische, pharmakologische Wirkort- und/ oder Wirkstofftestung:
- mit einem Sekundärträger (20), welcher einen elektrisch leitfähigen und festkörperartigen Elektrodenbereich (21) aufweist,
- mit einer Mehrzahl Primärträger (10), welche in unmittelbarer räumlicher Nachbarschaft des Sekundärträgers (20) angeordnet sind und welche zu einer elektrischen Aktion aktivierbare und biologische Einheiten (12), insbesondere Membranproteine, aufweisen, und
- mit einem wässrigen Messmedium (30), in welchem die Primärträger (10) und der Sekundärträger (20) angeordnet sind.
- wobei der Elektrodenbereich (21) gegenüber dem Messmedium (30) elektrisch isoliert ausgebildet ist.
- wobei der Elektrodenbereich (21) gegenüber den Primärträgern (10) und gegenüber den biologischen Einheiten (12) elektrisch isoliert ausgebildet ist,
- wobei als Primärträger (10) jeweils eine eukariontische Zelle, eine prokariontische Zelle, ein Bakterium, ein Virus oder Bestandteile, insbesondere Membranfragmente, oder Verbände davon in nativer Form oder in abgewandelter Form, insbesondere in gereinigter, mikrobiologisch und/oder molekularbiologisch geänderter Form, vorgesehen ist, oder
- wobei als Primärträger (10) jeweils ein Vesikel, ein Liposom oder eine mizelläre Struktur vorgesehen ist.
- wobei der Elektrodenbereich (21) mindestens eine elektrisch leitfähige Elektrode (26) aufweist,
- wobei ein elektrisch isolierender Isolationsbereich (24) vorgesehen ist,
- wobei durch den Isolationsbereich (24) die jeweilige Elektrode (26) vom Messmedium (30), von den Primärträgern (10) und von den biologischen Einheiten (12) elektrisch isoliert ist.
- wobei der Isolationsbereich (24) schichtartig ausgebildet ist.
- wobei der Isolationsbereich (24) zumindest zum Teil aus einer Abfolge von Monoschichten (24a, 24b) besteht.
- dass die Monoschichten (24a, 24b) als spontan selbstorganisierende Schichten ausgebildet sind.
- wobei als Unterschicht (24b) des Isolationsbereichs (24) eine Schicht aus einer organischen Thioverbindung als unterster und der Elektrode (26) zugewandter Bereich (24b) des Isolationsbereichs (24) vorgesehen ist, vorzugsweise aus einem langkettigen Alkanthiol, insbesondere aus Oktadekanthiol, und
- wobei als Oberschicht (24a) des Isolationsbereichs (24) eine Schicht aus einer amphiphilen organischen Verbindung, insbesondere aus einem Lipid, als oberster und von der Elektrode (26) abgewandter Bereich oder Oberflächenbereich (24a) des Isolationsbereichs (24) vorgesehen ist.

2. Sensoranordnung nach Anspruch 1.
**dadurch gekennzeichnet,**
**dass** der die Elektrode (26) isolierende und abdeckende Bereich des Isolationsbereichs (24) eine Membranstruktur (SSM) mit einer Fläche von etwa A ≈ 0.1 - 50 mm² und mit einer spezifischen elektrischen Leitfähigkeit von etwa Gₘ ≈ 1 - 100 nS/cm² und/oder mit einer spezifischen Kapazität von etwa Cₘ ≈ 10 - 1000 nF/cm² aufweist.

3. Sensoranordnung nach einem der Ansprüche 1 oder 2.
**dadurch gekennzeichnet.**
- **dass** die Elektrode (26) ein metallisches Material aufweist, insbesondere ein Edelmetall, vorzugsweise Gold, oder
- **dass** die Elektrode (26) ein elektrisch leitfähiges Metalloxid aufweist, insbesondere Indiumzinnoxid.

4. Sensoranordnung nach einem der vorangehenden Ansprüche,
**dadurch gekennzeichnet,**
**dass** die biologische Einheit (12) zu einer elektrogenen Ladungsträgerbewegung aktivierbar ausgebildet ist, insbesondere zu einem elektrogenen Ladungsträgertransport.

5. Sensoranordnung nach einem der vorangehenden Ansprüche,
**dadurch gekennzeichnet,**
**dass** als biologische Einheit (12) jeweils ein Membranprotein, insbesondere eine Ionenpumpe, ein Ionenkanal, ein Transporter, ein Rezeptor oder ein Bestandteil oder ein Verband davon vorgesehen ist.

6. Sensoranordnung nach einem der vorangehenden Ansprüche,
**dadurch gekennzeichnet,**
**dass** die biologische Einheit (12) in nativer Form oder in abgewandelter Form, insbesondere gereinigter, mikrobiologisch und/oder molekularbiologisch geänderter Form, vorgesehen ist.

7. Sensoranordnung nach einem der vorangehenden Ansprüche,
**dadurch gekennzeichnet,**
- **dass** die Oberfläche (10a) der Primärträger (10) und die Oberfläche des Sekundärträgers (20) entgegengesetzt polar oder geladen zueinander ausgebildet sind und/oder
- **dass** zwischen der Oberfläche (10a) der Primärträger (10) und der Oberfläche des Sekundärträgers (20) eine Ankopplung nach Art einer chemischen Bindung ausgebildet ist, insbesondere über eine His-Tag-Kopplung oder eine Streptavidin-Biotin-Kopplung.

8. Vorrichtung für die amperometrische und/oder potentiometrische, pharmakologische Wirkort- und/oder Wirkstofftestung:
- mit mindestens einem Messbereich (50), in welchem als Messsonde eine Sensoranordnung (1) nach einem der Ansprüche 1 bis 7 verwendet wird,
- mit einer Datenerfassungs-/Steuereinrichtung (40) welche zumindest zur Erfassung von Messdaten der Sensoranordnung (1) ausgebildet ist, und
- mit einer Austausch- und Mischeinrichtung (60), welche zum Verfügungstellen, Austausch, Mischen und Einstellen des Messmediums (30) ausgebildet ist.

9. Vorrichtung nach Anspruch 8.
**dadurch gekennzeichnet,**
- **dass** mittels der Austauscheinrichtung (60) über das Messmedium (30) Messbedingungen, insbesondere pharmakologische Bedingungen, definiert in kontinuierlicher Art und Weise einstellbar sind, vorzugsweise nach Art eines kontinuierlichen Fließsystems, und
- **dass** dabei mittels der Austauscheinrichtung (60) in der Nachbarschaft der Sensoranordnung (1) Fließ- und Strömungsgeschwindigkeiten von etwa v ≈ 0,1 - 2 m/s erzeugbar sind.

10. Vorrichtung nach einem der Ansprüche 8 oder 9,
**dadurch gekennzeichnet,**
**dass** eine Mehrzahl integrierter Sensoranordnungen (1), vorzugsweise in separaten, strömungsmäßig und elektrisch voneinander entkoppelten und unabhängigen Vertiefungsbereichen einer Mikroplatte oder Mikrotiterplatte vorgsehen ist, vorzugsweise um 8, 12, 96 Messkanäle auf einem Raster auszubilden.

11. Verfahren zur amperometrischen und/oder potentiometrischen, pharmakologischen Wirkort- und/oder Wirkstofftestung:
- bei welchem zu untersuchende biologische Einheiten (12) in mindestens einer Sensoranordnung (1) vorgesehen werden und
- bei welchem über die Sensoranordnung (1) aktivierte elektrische Aktionen der biologischen Einheiten (12) gemessen werden,
**dadurch gekennzeichnet,**
- **dass** eine Sensoranordnung (1) nach einem der Ansprüche 1 bis 7 oder
- **dass** eine Vorrichtung nach einem der Ansprüche 8 bis 10 verwendet wird.

12. Verfahren nach Anspruch 11.
**dadurch gekennzeichnet.**
- **dass** die Sensoranordnung (1) jeweils vom Messmedium (30) umströmt oder angeströmt wird und
- **dass** dabei eine Strömungs- und/oder Fließgeschwindigkeit des Messmediums (30) von etwa v ≈ 0.1 - 2 m/s verwendet wird.

13. Verfahren nach einem der Ansprüche 11 oder 12,
**dadurch gekennzeichnet,**
**dass** aufeinanderfolgend eine Mehrzahl von Tests durchgeführt wird durch aufeinanderfolgendes Austauschen des Messmediums (30), gegebenenfalls mit zwischengeschaltetem Waschen oder Spülen des Messbereichs (50).

14. Verfahren nach einem der Ansprüche 11 bis 13,
**dadurch gekennzeichnet,**
- **dass** ein Deorphaningprozess mit einer Mehrzahl von Tests durchgeführt wird und
- **dass** dabei als biologische Einheit (12) ein Produkt eines funktionsunbekannten Orphans verwendet wird, um dabei dessen Funktionalität aufzuklären.

## Claims

1. Sensor arrangement for pharmacological testing of an active site and/or active ingredient using amperometric and/or potentiometric means:
- with a secondary carrier (20) which has an electrically conductive and solid-like electrode area (21),
- with a plurality of primary carriers (10) which are located in the immediate spatial vicinity of the secondary carrier (20) and which have biological units (12), especially membrane proteins, which can be activated into electrical action,
- with an aqueous measurement medium (30) which contains the primary carriers (10) and the secondary carrier (20),
- wherein the electrode area (21) is made electrically insulated relative to the measurement medium (30),
- wherein the electrode area (21) is made electrically insulated relative to the primary carriers (10) and relative to the biological units (12),
- wherein the primary carrier (10) is one of a eukaryotic cell, a procaryotic cell, a bacterium, a virus, or components, especially membrane fragments, or associations thereof in native form or in altered form, especially in purified, microbiologically and/or molecular biologically altered form, or,
- wherein the primary carrier (10) is one of a vesicle, a liposome or a micellar structure,
- wherein the electrode area (21) has at least one electrically conductive electrode (26),
- wherein there is an electrically insulating insulation area (24),
- wherein the respective electrode (26) is electrically insulated by the insulation area (24) from the measurement medium (30), from the primary carriers (10) and from the biological units (12),
- wherein the insulation area (24) is made layer-like,
- wherein the insulation area (24) consists at least in part of a sequence of monolayers (24a, 24b),
- wherein the monolayers (24a, 24b) are made as spontaneously self-organizing layers.
- wherein an underlayer (24b) of the insulation area (24) is a layer of an organic thio compound as a lowermost region (24b) of the insulation area (24) facing the electrode (26), preferably of a long-chain alkane thiol, especially of an octadecane thiol, and
- wherein an upper layer (24a) of the insulation area (24) is a layer of an amphiphilic organic compound, especially of a lipid, as the uppermost region or surface area (24a) of the insulation area (24) facing away from the electrode (26).

2. Sensor arrangement according to claim 1, wherein the region of the insulation area (24) which covers and insulates the electrode (26) has a membrane structure (SSM) with an area of roughly A = 0.1 - 50 mm² and with a specific electrical conductivity of roughly Gₘ = 1 - 100 nS/cm² and/or with a specific electrical capacitance of roughly Cₘ = 10 - 1000 nF/cm².

3. Sensor arrangement according to any one of the preceding claims 1 or 2,
- wherein the electrode (26) has a metallic material, especially a precious metal, preferably gold, or
- wherein the electrode (26) has an electrically conductive metal oxide, especially indium tin oxide.

4. Sensor arrangement according to any one of the preceding claims,
wherein the biological unit (12) is activatable to electrogenic charge carrier movement, especially to electrogenic charge carrier transport.

5. Sensor arrangement according to any one of the preceding claims,
wherein the biological unit (12) is one of a membrane protein, especially an ion pump, an ion channel, a transporter, a receptor or a component or an association thereof.

6. Sensor arrangement according to any one of the preceding claims,
wherein the biological unit (12) is provided in native form or in altered form, especially in purified, microbiologically and/or molecular biologically altered form.

7. Sensor arrangement according to any one of the preceding claims,
- wherein the surface (10a) of the primary carrier (10) and the surface of the secondary carrier (20) are made polarly opposite or oppositely charged to one another and/or
- wherein between the surface (10a) of the primary carrier (10) and the surface of the secondary carrier (20) coupling in the manner of a chemical bond is formed, especially via his-tag coupling or streptavidin-biotin coupling.

8. Device for pharmacological testing of an active site and/or active ingredient using amperometric and/or potentiometric means:
- with at least one measurement area (50) in which there is employed a sensor arrangement (1) as the measurement probe according to any one of claims 1 to 7.
- with a data acquisition/control means (40) which is made at least for acquiring the measurement data of the sensor arrangement (1) and
- with an exchange and mixing means (60) which is made for making available, exchanging, mixing and/or adjusting the measurement medium (30).

9. Device according to claim 8,
- wherein by the exchange means (60) via the measurement medium (30) the measurement conditions, especially the pharmacological conditions, can be adjusted in a defined manner, especially in the manner of a continuous flow system, and
- wherein a flow velocity of roughly v = 0.1 - 2 m/s can be produced by the exchange means (60) in the vicinity of the sensor arrangement (1).

10. Device as claimed in one of claims 8 or 9,
wherein a plurality of integrated sensor arrangements (1) is provided, preferably in separate and independent recess areas of a microplate or microtiter plate, which areas are decoupled from one another in terms of electricity and flow, preferably in order to form 8, 12, 96 measurement channels on a grid.

11. Process for pharmacological testing of an active site and/or active ingredient using amperometric and/or potentiometric means:
- in which biological units (12) to be studied are provided in at least one sensor arrangement (1) and
- in which electrical actions of the biological units (12) which have been activated are measured via the sensor arrangement (1),
- wherein a sensor arrangement (1) as claimed in one of claims 1 to 7 is employed or
- wherein a device as claimed in one of claims 8 to 10 is employed.

12. Process according to claim 11,
- wherein the measurement medium (30) flows past or against the sensor arrangement (1) and
- wherein a flow velocity of the measurement medium (30) of roughly v = 0.1 - 2 m/s is used.

13. Process according to any one of the claims 11 or 12,
wherein a plurality of tests is carried out in succession by successive exchange of the measurement medium (30), optionally with interposed washing or flushing process of the measurement area (50).

14. Process according to any one of claims 11 to 13,
- wherein a deorphaning process is carried out with a plurality of tests and
- wherein a product of an orphan of unknown function is used as the biological unit (12) in order to clarify its functionality.

## Revendications

1. Ensemble de détection pour contrôler des sites actifs et/ou des substances actives d'un point de vue pharmacologique à l'aide d'un ampèremètre et/ou d'un potentiomètre :
- avec un porteur secondaire (20) qui comporte une région d'électrode (21) conductrice électriquement et de type solide,
- avec plusieurs porteurs primaires (10) qui sont disposés dans le voisinage spatial direct du porteur secondaire (20) et qui comportent des unités (12) biologiques et activables pour une action électrique, en particulier des protéines membranaires, et
- avec un milieu de mesure aqueux (30) dans lequel sont disposés les porteurs primaires (10) et le porteur secondaire (20),
- dans lequel la région d'électrode (21) est conçue avec une isolation électrique par rapport au milieu de mesure (30),
- dans lequel la région d'électrode (21) est conçue avec une isolation électrique par rapport aux porteurs primaires (10) et par rapport aux unités biologiques (12),
- dans lequel, en tant que porteur primaire (10), il est prévu à chaque fois une cellule eucaryotique, une cellule procaryotique, une bactérie, un virus ou des éléments, en particulier des fragments de membrane, ou des assemblages de ces derniers sous une forme native ou sous une forme modifiée, en particulier sous une forme nettoyée, modifiée de manière microbiologique et/ou moléculaire biologique, ou
- dans lequel, en tant que porteur primaire (10), il est prévu à chaque fois une vésicule, un liposome ou une structure micellaire,
- dans lequel la région d'électrode (21) comporte au moins une électrode conductrice électriquement (26),
- dans lequel il est prévu une région d'isolation (24) isolante électriquement,
- dans lequel ladite électrode (2B) est isolée électriquement par la région d'isolation (24) du milieu de mesure (30), des porteurs primaires (10) et des unités biologiques (12),
- dans lequel la région d'isolation (24) est conçue avec plusieurs couches,
- dans lequel la région d'isolation (24) se compose au moins en partie d'une suite de monocouches (24a, 24b),
- les monocouches (24a, 24b) étant conçues sous la forme de couches s'organisant automatiquement de manière spontanée.
- dans lequel, en tant que sous-couche (24b) de la région d'isolation (24), il est prévu une couche faite d'une thio-liaison organique en tant que région (24b), la plus basse et tournée vers l'électrode (26), de la région d'isolation (24), de préférence faite d'un alcantiol à longue chaîne, en particulier d'un octadecanethiol, et
- dans lequel, en tant que couche supérieure (24a) de la région d'isolation (24), il est prévu une couche faite d'une liaison organique amphiphile, en particulier faite d'un lipide, en tant que région ou région de surface (24a), la plus haute et opposée à l'électrode (26), de la région d'isolation (24).

2. Ensemble de détection selon la revendication 1,
**caractérisé en ce que** la région, recouvrant et isolant l'électrode (26), de la région d'isolation (24) comporte une structure de membrane (SSM) avec une surface A d'environ 0,1 à 50 mm² et avec une conductivité électrique spécifique Gₘ d'environ 1 à 100 nS/cm² et/ou avec une capacité spécifique Cₘ d'environ 10 à 1000 nF/cm².

3. Ensemble de détection selon l'une des revendications 1 ou 2, **caractérisé en ce que :**
- l'électrode (26) comporte un matériau métallique, en particulier un métal précieux, de préférence de l'or, ou
- l'électrode (26) comporte un oxyde de métal conducteur électriquement, en particulier un oxyde d'indium-étain.

4. Ensemble de détection selon l'une quelconque des revendications précédentes, **caractérisé en ce que** l'unité biologique (12) est conçue de manière activable pour un mouvement électrogène de porteurs de charge, en particulier pour un transport électrogène de porteurs de charge.

5. Ensemble de détection selon l'une quelconque des revendications précédentes, **caractérisé en ce que,** en tant qu'unité biologique (12), il est prévu à chaque fois une protéine membranaire, en particulier une pompe à ions, un canal ionique, un transporteur ou un récepteur ou un élément ou un assemblage de ceux-ci.

6. Ensemble de détection selon l'une quelconque des revendications précédentes, **caractérisé en ce que** l'unité biologique (12) est prévue sous une forme native ou sous une forme modifiée, en particulier sous une forme modifiée nettoyée, modifiée de manière microbiologique et/ou moléculaire biologique.

7. Ensemble de détection selon l'une quelconque des revendications précédentes, **caractérisé en ce que :**
- la surface (10a) des porteurs primaires (10) et la surface du porteur secondaire (20) sont conçues avec une charge ou une polarité opposée l'une par rapport à l'autre et/ou
- un couplage est réalisé entre la surface (10a des porteurs primaires (10) et la surface du porteur secondaire (20) sous forme d'une liaison chimique, en particulier par un couplage His-Tag ou un couplage streptavidin-biotin.

8. Dispositif pour le contrôle de sites actifs et/ou de substances actives d'un point de vue pharmacologique à l'aide d'un ampèremètre et/ou d'un potentiomètre comportant :
- au moins une plage de mesure (50) dans laquelle un ensemble de détection (1) selon l'une des revendications 1 à 7 sert de sonde de mesure,
- un système de commande et de collecte de données (40) qui est conçu au moins pour collecter des données de mesure de l'ensemble de détection (1), et
- un système d'échange et de mélange (60) qui est conçu pour mettre à disposition, échanger, mélanger et ajuster le milieu de mesure (30).

9. Dispositif selon la revendication 8, **caractérisé en ce que :**
- au moyen du système d'échange (60) sur le milieu de mesure (30), des conditions de mesure, en particulier des conditions pharmacologiques, définies d'une manière continuelle, peuvent être réglées, de préférence à la manière d'un système de fluage continu, et
- des vitesses de fluage et d'écoulement v d'environ 0,1 à 2 m/s peuvent être réalisées au moyen du système d'échange (60) dans le voisinage de l'ensemble de détection (1) .

10. Dispositif selon l'une des revendications 8 ou 9, **caractérisé en ce qu'**il est prévu plusieurs ensembles de détection (1) intégrés, de préférence dans des régions évidées et indépendantes, d'une microplaque ou d'une plaque microtitre, séparées, découplées les unes des autres de manière électrique et par rapport à l'écoulement, de préférence pour former 8, 12, 96 canaux de mesure dans un réseau.

11. Procédé pour le contrôle de sites actifs et/ou de substances actives d'un point de vue pharmacologique à l'aide d'un ampèremètre et/ou d'un potentiomètre :
- avec lequel des unités biologiques (12) à analyser sont prévues dans au moins un ensemble de détection (1) et
- avec lequel des actions électriques des unités biologiques (12), activées par l'ensemble de détection (1), sont mesurées,
**caractérisé en ce que .**
- le procédé emploie un ensemble de détection (1) selon l'une des revendications 1 à 7 ou
- le procédé emploie un dispositif selon l'une des revendications 8 à 10.

12. Procédé selon la revendication 11, **caractérisé en ce que**
- le milieu de mesure (30) s'écoule autour ou au travers de l'ensemble de détection (1) et
- une vitesse d'écoulement et/ou de fluage v du milieu de mesure (30) d'environ 0,1 à 2 m/s est employée.

13. Procédé selon l'une des revendications 11 ou 12, **caractérisé en ce que** plusieurs tests se succédant sont exécutés par un échange successif du milieu de mesure (30), le cas échéant en intercalant un lavage ou un rinçage de la région de mesure (50).

14. Procédé selon l'une des revendications 11 à 13, **caractérisé en ce que :**
- un processus de détection de ligands naturels est effectué avec plusieurs contrôles et
- un produit d'un orphelin ayant une fonction inconnue sert d'unité biologique (12) afin d'en déterminer la fonctionnalité.
